# EUROPEAN PATENT SPECIFICATION

(11) **EP 3 116 995 B1**
(45) Date of publication and mention of the grant of the patent: **12.09.2018**
(21) Application number: 15714426.2
(22) Date of filing: 06.03.2015
(51) Int. Cl.: C12N 15/10

(54) **PURIFICATION OF DNA-CONJUGATE PRODUCTS**
REINIGUNG VON DNA-KONJUGATPRODUKTEN
PURIFICATION DE PRODUITS CONJUGUÉS D'ADN

(30) Priority: 14.03.2014 GB 201404552
(43) Date of publication of application: 18.01.2017
(73) Proprietor: Philochem AG, 8112 Otelfingen (CH)
(72) Inventor: FRANZINI, Raphael, Salt Lake City, UT 84112 (US); SAMAIN, Florent, CH-8105 Regensdorf (CH); NERI, Dario, CH-8107 Buchs (CH)
(74) Representative: Sutcliffe, Nicholas Robert
(86) International application number: PCT/EP2015/054768
(87) International publication number: WO 2015/135856

(56) References cited:
- WO-A1-2010/094036
- WO-A1-2013/036810
- WO-A2-2004/016767
- WO-A2-2004/039825
- WO-A2-2005/058479
- WO-A2-2009/077173

## Description

This invention relates to methods for purifying the products of nucleic acid conjugation reactions from unreacted reactants, in particular, in the production of purified nucleic acid-conjugates for nucleic acid encoded chemical libraries.

Nucleic acid encoded chemical libraries are collections of chemical moieties covalently linked to identifier oligonucleotides encoding the identity of the chemical moieties. The members of nucleic acid encoded chemical libraries display pharmacophores made up of one or more chemical moieties (also called "building blocks"). These chemical libraries can be used to identify pharmacophores which are candidate binding agents or have improved characteristics, for example improved binding. DNA-encoded chemical libraries (DEL) are a particular type of nucleic acid encoded chemical libraries for which the nucleic acids is deoxyribonucleic acid (DNA).

Diverse populations of pharmacophores are produced by different combinations of chemical moieties. Each library member is tagged with a nucleic acid strand comprising nucleotide sequences that encode the chemical moieties that constitute the pharmacophore that is displayed by the member. This allows rapid identification of selected library members during screening.

DNA-encoded chemical library (DEL) technology allows the synthesis and screening of pharmacophores libraries of unprecedented size. DEL represents an advance in medicinal chemistry, which bridges the fields of combinatorial chemistry and affinity selection techniques (e.g., phage display, mRNA display) and promises to revolutionise the drug discovery field and to reshape the way pharmaceutically relevant compounds are traditionally discovered. Recent advances in ultrahigh-throughput nucleic acid sequencing (e.g., Illumina sequencing, SOLiD technology, etc.) indicate that it should be possible to sequence even billions of sequence tags per sequencing run. With suitable synthetic and encoding procedures, it should be possible to construct, perform selections, and decode nucleic acid-encoded libraries comprising multiple building blocks and containing millions to billions of chemical compounds [1].

DNA encoded chemical libraries may display pharmacophores that are formed of chemically linked chemical moieties that are all attached to a single strand of nucleic acid ("single pharmacophore libraries") or pharmacophores that are formed of chemical moieties that are attached to two different strands of nucleic acid hybridised together, one or more chemical moieties being attached to each strand ("dual pharmacophore libraries").

DNA-encoded chemical libraries were proposed first by Sydney Brenner and Richard Lerner in 1992 ([2]; US 5,573,905; WO93/20242). These authors postulated the alternating stepwise synthesis of a polymer (e.g. a peptide) and an oligonucleotide sequence (serving as a coding sequence) on a common linker (e.g. a bead) in split and pool cycles. After affinity capture on a target protein, the population of identifier oligonucleotides of the selected library members would be amplified by PCR and, in theory, used for enrichment of the bound molecules by serial hybridisation steps to a subset of the library. In principle, the affinity-capture procedure could be repeated, possibly resulting in a further enrichment of the active library members. Finally, the structures of the chemical entities would be decoded by cloning and sequencing the PCR products. It was postulated that encoding procedures could be implemented by a variety of methods, including chemical synthesis, DNA polymerization or ligation of DNA fragments [2].

The feasibility of the orthogonal, solid-phase synthesis of peptides and oligonucleotides was demonstrated by attaching a test peptide (the pentapeptide leucine enkephalin) and an encoding identifier oligonucleotide onto controlled-pore glass beads [3]. The peptide bound to a specific antibody and the corresponding DNA coding tag was amplified by PCR. The technology was used to construct a collection of ∼10⁶ heptapeptide sequences and their corresponding identifier oligonucleotide tags on beads. The library was incubated with a fluorescently labelled anti-leucine enkephalin antibody, and binders were selected successfully by fluorescent-assisted cell sorting [4]. In their original paper, Brenner and Lerner suggested that the alternate synthesis of chemical compounds and oligonucleotides could also be implemented in the absence of beads. The use of enzyme catalysed ligation of coding DNA fragments is now established in the field (US 2006-0246450; WO 02/103008; WO2004/013070; WO2004/074429; WO2007/062664 and WO2005/058479).

Various methods of generating DNA-encoded chemical libraries have been described in the art (see for example [1, 2, 5-9]; WO2009/077173; WO2003/076943; WO2013/036810; WO2010/094036; WO2004/016767; WO2004/039825). Standard strategies for encoding DNA-encoded chemical libraries based on two sets of building blocks involve the stepwise ligation of double-stranded DNA-fragments (containing a code for the unambiguous identification of each building block) after each addition of a chemical moiety in the library ([2], US 2006-0246450; WO 02/103008; WO2004/013070; WO2004/074429; WO2007/062664 and WO 2005/058479). Other methods of encoding include polymerase extension of single stranded DNAs with overlapping sequences, enzymatic ligation, and chemical ligation. In other words, the insertion of a chemical building block in a nascent chemical structure is associated with the ligation of a DNA-fragment that serves as code for that building block. The final code of the complete molecule is provided by the sum of the codes, corresponding to the individual building blocks.

The development of methods for the purification of nucleic acid conjugates is useful in the preparation of nucleic acid chemical libraries with a high degree of purity.

A first aspect of the invention provides a method of producing a purified population of nucleic acid conjugate products comprising;
providing reaction members that comprise nucleic acid conjugate reaction products and nucleic acid or nucleic acid conjugate reactants, wherein said reactants comprise a first reactive group,
contacting the reaction members with a capping molecule comprising a capture group, wherein the capping molecule reacts with the first reactive group to form a covalent bond, thereby attaching the capture group to nucleic acid or nucleic acid conjugate reactants in reaction members,
removing the nucleic acid or nucleic acid conjugate reactants from the reaction members using a binding member that binds the capture group,
thereby producing a purified population of nucleic acid conjugate products.

The reaction members comprise unreacted reactants and reaction products produced by a reaction between reactants. Generally the reaction members will comprise nucleic acid conjugate products, which are desired, and unreacted nucleic acid or nucleic acid conjugate reactants, which it is desirable to isolate from the products.

The reaction members may be provided by reacting a population of nucleic acid or nucleic acid conjugate reactants having a first reactive group with a population of chemical moieties that react with the first reactive group to produce reaction members comprising nucleic acid conjugate products that contain the chemical moiety and unreacted nucleic acid or nucleic acid conjugate reactants.

The reaction occurs between corresponding reactive groups on each of the reactants and results in the formation of a bond, generally a covalent bond, between the reactants. The reaction between the reactants produces a population of reaction members.

The reaction between the reactants may not go to completion (i.e. the reaction may be incomplete). Thus, the reaction members comprise both the desired reaction products and unreacted reactants.

Optionally, the methods described herein may further comprise:
(a) reacting the purified population of nucleic acid conjugates with a population of additional chemical moieties reactive with the chemical moiety of the nucleic acid conjugate, to produce reaction members comprising nucleic acid conjugate products comprising the additional chemical moiety and nucleic acid conjugate reactants,
(b) contacting the mixture with a capping molecule that comprises a capture group, wherein the capping group reacts with the chemical moiety of the nucleic acid conjugate reactants in the reaction members to form covalent bonds, thereby attaching the capture group to nucleic acid conjugate reactants, and,
(c) removing the unreacted nucleic acid conjugate reactants from the reaction members using a binding member that binds the capture group,
thereby producing a purified population of nucleic acid conjugate products comprising the additional chemical moiety.

Steps (a) to (c) may be repeated one or more times to produce a purified population of nucleic acid conjugate products comprising two or more additional chemical moieties.

Preferably, the nucleic acid is DNA.

A purified population contains an increased proportion of desired reaction products compared to unreacted reactants, relative to the unpurified population. For example, a purified population of reaction products may comprise at least 50%, 55%, 60%, 65%, 70%, 75%, 80%, 85%, 90%, 95%, 96%, 97%, 98% or 99% nucleic acid conjugate products compared to unreacted reactants.

Before purification as described herein the reaction members comprise both nucleic acid conjugate products and nucleic acid or nucleic acid conjugate reactants. The nucleic acid or nucleic acid conjugate reactants in the reaction members are unreacted reactants. In order to increase the purity of the nucleic acid conjugate products, it is desirable to remove the unreacted reactants.

A nucleic acid is a molecule formed from a plurality of nucleotide monomers. The nucleic acid may be single stranded or double stranded. The nucleic acid may be a single stranded nucleic acid, for example a single stranded DNA strand or a DNA conjugate comprising single stranded DNA. The nucleic acid may be a double stranded nucleic acid, for example a double stranded DNA strand or a DNA conjugate comprising double stranded DNA.

Generally, the nucleic acid is less than 1 kilobase (or kilobase pairs) in length, preferably less than 500 bases or base pairs in length. The nucleic acid may be from 50-500 base pairs in length, 100-400, 100-300, 100-200 base pairs in length or any combination of these ranges. Preferably the nucleic acid is from about 100-200 base pairs in length.

The nucleic acid may comprise DNA, RNA, PNA (peptide nucleic acid) LNA (locked nucleic acid), 2'-fluoro DNA, 2'-methoxy DNA or HNA (hexose nucleic acid) bases. Preferably the nucleic acid is DNA.

A nucleic acid conjugate comprises a nucleic acid molecule linked (conjugated) to one or more chemical moieties.

The nucleic acid conjugate may be a product of a conjugation reaction (nucleic acid conjugate product). Nucleic acid conjugate products are formed when two or more reactants are reacted together. For example, nucleic acid conjugate products may be formed when a nucleic acid is conjugated to a chemical moiety or when a further chemical moiety is conjugated to a nucleic acid conjugate.

The nucleic acid conjugate may be a reactant (nucleic acid conjugate reactant), which is reacted with a further reactant to form a reaction product. For example, a nucleic acid conjugate reactant may be conjugated with one or more chemical moieties to form a nucleic acid conjugate product.

Nucleic acid conjugate reactants may comprise a nucleic acid strand linked to n chemical moieties, where n is 1 to 10, (for example 1, 2,3,4,5,6,7,8,9 or 10 chemical moieties), and the nucleic acid conjugate products may comprise a nucleic acid molecule linked to n+1 chemical moieties.

The nucleic acid conjugate may be a member of a nucleic acid chemical library, including nucleic acid-encoded chemical libraries such as a DNA-encoded chemical library, or may be a nascent chemical library member. The chemical moieties linked to the nucleic acid or nucleic acid conjugate may form a pharmacophore for screening and selection on a target of interest. Generally the chemical moieties in the pharmacophore will have different structures to each other.

DNA chemical libraries and DNA-encoded chemical libraries (DELs) are well-known in the art.

Nucleic acid reactants may comprise nucleic acid strands having a first reactive group.

In some embodiments, where the reactant is a nucleic acid strand, the reactive group may be preferably located at a terminal of the nucleic acid strand. For example, the first reactive group may be located at the 5' or 3' terminal of the nucleic acid strand.

The terminal of the nucleic acid strand is not limited to the nucleotide at the very end of the strand (the terminal nucleic acid) but includes a number of nucleotides located adjacent to the terminal nucleotide. For example the terminal of the nucleic acid strand generally comprises the 1, 2, 3, 4, 5, 6, 7, 8, 9, or 10 nucleotides located adjacent to the terminal nucleic acid.

In other embodiments, for example where the reactant is a nucleic acid strand which forms a hairpin structure (e.g. hairpin DNA or RNA), the reactive group may be located outside a terminal of the nucleic acid strand, for example at the centre.

Nucleic acid-conjugate reactants may comprise nucleic acid conjugate reactants having a first reactive group.

Where the reaction members comprise nucleic acid conjugate reactants having a first reactive group, the nucleic acid conjugate reactants may comprise a nucleic acid strand conjugated to at least one chemical moiety. The conjugated chemical moiety may comprise the first reactive group.

The reaction members may also comprise chemical moieties having a first reactive group. Unreacted chemical moieties are generally removed from the reaction members by existing purification techniques known in the art such as HPLC, ethanol precipitation, gel electrophoresis, ion exchange chromatography, affinity purification (for example on a C12 resin but without a HPLC). In the case of synthesis on solid phase or DNAs immobilized on (ion exchange) resins, the unreacted small molecules and side products may be removed by washing the solid support.

This is because the chemical moiety is generally small and easily separated from the nucleic acid or nucleic acid conjugate reactants. However, unreacted chemical moieties may also be removed using a suitable capping molecule in the same way that unreacted nucleic acid or nucleic acid conjugate reactants are removed. Unreacted chemical moieties may be removed from the reaction members before or after the purification as described herein.

The unreacted reactants (e.g. unreacted nucleic acid strands or nucleic acid conjugates) may be removed by contacting the reaction members with a capping molecule comprising a capture group. The capping molecule reacts with a first reactive group located on the unreacted reactants. This links the capping molecule to the reactants. The reactants may then be removed from the reaction members using a binding member which binds the capture group. Removing the unreacted reactants from the reaction members increases the purity of the reaction products, which do not bind the capping molecule and therefore do not bind the binding member.

In the reaction products, the first reactive group has already been reacted to form the products. This means that the capping molecule is unable to react with the first reactive group in the reaction products.

A reactive group is a chemical group that is capable of forming a bond, preferably a covalent bond with a further reactive group.

One or more reactive groups are located on the reactants. For example, one or more reactive groups may be located on a nucleic acid strand or nucleic acid conjugate.

A reaction between reactive groups located on different reactants forms a bond which links the reactants together to form a reaction product. The formation of a reaction product means that the reactive groups located on the reactants will not be available to react with the capping molecule. However, unreacted reactants will still comprise a reactive group which is capable of reacting with the capping molecule. Thus, during purification as described herein, the capping molecule can be used to remove unreacted reactants from the reaction members, leaving the reaction products.

Where the nucleic acid strand or nucleic acid conjugate comprises a chemical moiety, the chemical moiety may comprise a first reactive group.

The chemical moiety may be a reactant which is reacted with first reactive group on a nucleic acid or nucleic acid conjugate to produce a nucleic acid conjugate product.

The chemical moiety may comprise one or more further reactive groups which may participate in subsequent reactions. Such further reactive groups may be protected, or may be orthogonal to the first reactive group, as described herein. The further reactive groups may be used to link a further chemical moiety to a nucleic acid conjugate comprising the chemical moiety. Thus, multiple chemical moieties may be added to a nucleic acid conjugate.

The chemical moiety and capping molecule are both capable of reacting with the same functional group on the reactants. Thus, any reactants which have not reacted with the chemical moiety can be removed by reacting the functional group with the capping molecule.

The reactive groups located on the chemical moiety and capping molecule may be the same. Alternatively, the reactive groups may be different, but still capable of reacting with the same reactive group (e.g. because they have the same chemistry).

Suitable reactive groups are well-known in the art. For example, the reactants may comprise a reactive group which is an amine and the chemical moiety and capping molecule may comprise a reactive group which is an activated carboxylic acid-derivative, or vice versa.
Here, the reactive group on the chemical moiety and capping molecule is the same.

Alternatively, the reactive group on the chemical moiety and capping molecule may be different. For example, one of the reactive groups may be an alkoxyamine or 1, 2-mercaptoamine and the other may be an amine. The reactive group on the reactants may then be an aldehyde.

Alternatively, one of the reactive groups may be a nucleophilic group, such as thiol or phosphorothioate, and the other may be a primary amine. The reactive group on the reactants may then be a chloroacetyl group.

When a reactant comprises more than one reactive group, any reactive groups which are not required to participate in a particular reaction may be protected with a suitable protecting group. This prevents any reactions from occurring at that reactive group. The protected reactive groups may later be deprotected so that they may participate in a reaction. Protective groups and techniques for protecting and deprotecting reactive groups are well known in the art, (see for example, [10]) as is explained further below.

In addition to protecting the reactive groups with a protective group, a further reactive group may be orthogonal to the first reactive group to prevent them from reacting with the chemical moiety or capping molecule. Orthogonal reactive groups have sufficiently different chemistries so that they do not react with the same further reactive groups.

Because reactants may comprise more than one reactive group, the reaction product may also comprise one or more further reactive groups. These reactive groups may be been protected during the formation of the reaction product and may subsequently be de-protected, so that the reaction product can participate in a further reaction. Alternatively, these further reactive groups may be orthogonal to the first reactive group.

A protecting group can be used to protect the functional groups which are not required to participate in a particular reaction. Protecting a functional group prevents it from reacting.

The choice of a protective group depends on the functional group which is to be protected. Suitable protective groups and techniques for deprotecting are well known in the art (see, e.g. [10]).

Suitable protecting groups include a boc protecting group (tert-Butyl carbamates) and an f-moc protecting group (9-fluorenylmethyloxycarbonyl).

Further suitable groups include 4-methyltrityl (Mtt), 4-methoxytrityl (Mmt), 4,4'-dimethoxytrityl (all can be used for -OH and NH2 groups and are protected, for example, with 3% TCA in DCM), 4-nitroveratryloxy carbonyl (NVOC, for amines, deprotection by UV-light), allyloxy carbonyl (Alloc, deprotection with palladium(0)), N-(1-(4,4-dimethyl-2,6-dioxocyclohexylidene)ethyl) (DDE, hydrazinolysis), cyclic acetals (for aldehydes, removal with acidic water). Further examples of protective groups are known in the art, see for example [10].

A protected reactive group may be de-protected so that it can then participate in a further reaction.

Techniques for de-protection are well known in the art. For example a boc group may be removed by trifluoroacetic acid (TFA) and an f-moc group may be removed using a secondary amine base like piperidine.

A binding member is used to remove the unreacted and capped reactants from the reaction members. The binding member binds to the capture group on the capping molecule. When the unreacted reactants are attached to the capping molecule, contacting the reactants with the binding member will bind the unreacted reactants to the binding member, through the binding between the capture group and binding member. This binding may be covalent (for example an azide tagged capping molecules could react with strained alkynes on a solid support) or non-covalent. The binding is generally non-covalent as explained below.

In some embodiments, the binding member is immobilised, for example on a solid support.

Solid supports are generally well known in the art and include beads, columns, arrays, multiwell plates, resins, polymers or other surface substrates.

Suitable solid supports are well known in the art and include cross-linked dextran such as Sephadex™ (Pharmacia Fine Chemicals (Piscataway, N.J.); agarose, borosilicate, polystyrene or latex beads about 1 micron to about 5 millimeters in diameter, polyvinyl chloride, polystyrene, cross-linked polyacrylamide, nitrocellulose or nylon-based webs such as sheets, strips, paddles, plates multiwell plate wells and other insoluble matrices.

Binding members can be immobilized on solid supports by physical adsorption, covalent coupling or other methods that are well known in the art.

Selection of the immobilization method depends on parameters such as the reaction conditions needed for coupling between the capture group and binding member, and the type of reactive groups present.
When the binding member is immobilised, reactants binding to the immobilized binding member via the capture moiety can be readily separated from the nucleic acid conjugate products, which do not bind to the binding member and may, for example remain present in the liquid phase. In other words, the unreacted reactants are removed by selective immobilization.

In some embodiments, the binding member may comprise a biotin-binding protein. The biotin-binding protein may be streptavidin or avidin.

Preferably, when the binding member comprises a biotin-binding protein, the capture group comprises biotin or a biotin derivative, for example a biotin derivative as described below.

In other embodiments, the binding member may comprise biotin or a biotin derivative and the capture group may comprise a biotin-binding protein.

The capping molecule comprises a capture group which is capable of binding to the binding member. Nucleic acid or nucleic acid conjugate reactants which have reacted with the capping molecule (which attaches the reactants to the capping molecule) can be removed from the reaction products by their binding to the binding member.

The binding between the capture group and binding member is generally achieved through non-covalent intermolecular forces such as ionic bonds, hydrogen bonds and van der Waals forces, which are generally reversible. The binding may occur through covalent bonding, which is generally irreversible. Suitable covalent bonds are well known in the art.

Exemplary non-covalent binding pairs suitable for use as a capture group or binding member include the following high affinity pairs: biotin-biotin-binding protein, protein A-Fc receptor, ferritin-magnetic beads, antibody/immunogenic epitope, glutathione-S-transferase/glutathione, chitin-binding protein/chitin (e.g. chitin resin), maltose binding protein/amylose, polyhistidine/nickel or cobalt, and the like. Other suitable binding pairs which may be used in the capture group or binding member are well known in the art. Thus, for example, the capture group may be biotin and the binding member may be avidin or streptavidin, the capture group may be protein A and the binding member may be Fc receptor, the capture group may be ferritin and the binding member may be magnetic beads and the like, or vice versa. The binding member may be in the solid phase as is well known in the art.

Preferably, the capture group is biotin or a biotin derivative. Suitable biotin derivatives include desthiobiotin and/or iminobiotin and the like.

The capture group may be joined to the capping molecule directly or via a linker. For example, when the capture group is biotin, the biotin may be joined to the reactive group by a linker. Suitable linkers are well known in the art.

Preferably, the capping molecule may consist or comprise biotin isobutyloxy anhydride (compound A) or biotin N-hydroxysuccinimidyl ester (compound B): 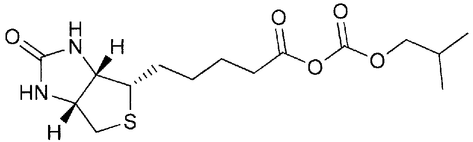 Compound A 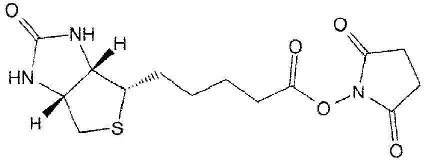 Compound B

Further exemplary capping molecules include activated esters, including pentafluorophenoxy, symmetric and asymmetric anhydrides. Other amine-reactive groups could be used in principle as well. The activated esters can be formed prior to the capping reaction or in situ by the addition of an activating agent. Methods for forming activated esters are known in the art.

Optionally, the purity of the population of nucleic acid conjugate products may be further increased by repeating the steps of:
contacting the reaction members with a capping molecule comprising a capture group, such that the capping molecule covalently binds to unreacted nucleic acid or nucleic acid conjugate reactants, and, removing the reactants using a binding member that binds to the capture group. These steps may be repeated one or more times.

Optionally, one or more purification steps may be carried out to remove unreacted chemical moieties. Chemical moieties may be removed by, for example, washing if the DNA is attached to a solid support during synthesis or alternatively by ethanol precipitation, extraction (e.g., chloroform) or elution from a C12-cartridge.

The present method generally avoids the need for additional HPLC purification.

The chemical moiety may be any moiety of interest. Suitable chemical moieties include small organic molecules, amino acid residues or other amino-containing moieties (optionally with appropriate amino protection); and peptides or globular proteins (including antibody domains). Where a reactant or product comprises more than one chemical moiety, the chemical moieties may be different (i.e. not identical in structure) from each other, or may be the same.

In some embodiments, a chemical moiety may have a molecular weight of 300 Da or less, for example about 100 to 300 Da.

Suitable populations of chemical moieties which may be linked to nucleic acids are well known in the art (see [1] to [9]).

A chemical moiety may be conjugated to the nucleic acid strand directly or indirectly, for example via a linker. Suitable linkers, such as alkyl chains or polyethylene glycol (PEG), are well known in the art.

Generally the addition of chemical moieties to a nucleic acid is sequential, so that in a first reaction, a first chemical moiety is conjugated to a nucleic acid strand to form a nucleic acid conjugate product. In a further reaction, a second chemical moiety may be conjugated to the nucleic acid conjugate to form a nucleic acid conjugate product. The further reaction may be repeated to add further chemical moieties to the nucleic acid conjugate product.

In some embodiments, in a first reaction, a first chemical moiety is conjugated to a nucleic acid conjugate to form a nucleic acid conjugate product. In a further reaction, a second chemical moiety may be conjugated to the nucleic acid conjugate product to form a further nucleic acid conjugate product. The second reaction may be repeated to add further chemical moieties to the nucleic acid conjugate product.

The sequential addition of chemical moieties may be part of a split and pool method which are well known in the art, see for example [1], [8] and [9].

Where one or more chemical moieties are conjugated to a nucleic acid strand, each chemical moiety may be conjugated to the nucleic acid strand, or one or more of the chemical moieties may be conjugated to one or more other chemical moieties.

Chemical moieties may be coupled to a nucleic acid strand via other chemical moieties. For example, each of the chemical moieties coupled to a nucleic acid strand may be covalently bonded to other chemical moieties and one of the chemical moieties may be coupled to the nucleic acid strand. Suitable methods for covalently bonding chemical moieties are well known in the art.

Where the chemical moieties form a pharmacophore, the pharmacophore may be formed from a single compound comprising the covalently bound chemical moieties coupled to a nucleic acid strand. In other embodiments, the pharmacophore may be formed from two or more chemical moieties which are covalently bonded to each other, and a further one or more chemical moieties which are not covalently bonded to the covalently bonded moieties.

For example, two or more chemical moieties may be covalently linked together, and to the nucleic acid strand, and a further one or more chemical moieties may be independently linked to the nucleic acid strand. This may occur when the two or more chemical moieties and further one or more chemical moieties are linked to a nucleic acid strand through different reactive groups on the nucleic acid strand. One or more reactive groups on the nucleic acid strand may be protected or orthogonal, as described herein, to prevent an undesired reaction occurring.

A nucleic acid strand or nucleic acid conjugate may comprise a first reactive group. In a nucleic acid conjugate comprising a nucleic acid strand conjugated to at least one chemical moiety, the conjugated chemical moiety may comprise a first reactive group. A further chemical moiety which reacts with the first reactive group may be reacted with the first reactive group on the nucleic acid strand or conjugated chemical moiety to produce reaction members comprising nucleic acid conjugate products that contain the chemical moiety.

In a further aspect, the present invention provides a method of making a nucleic acid chemical library comprising:
(a) providing a population of nucleic acid strands, each strand comprising a first reactive group,
(b) reacting the nucleic acid strands with a diverse population of chemical moieties capable of reacting with the first reactive group to produce reaction members comprising a diverse population of nucleic acid conjugates that comprise nucleic acid strands conjugated to chemical moieties,
(c) contacting the reaction members with a capping molecule that comprises a capture group, wherein the capping molecule reacts with the first reactive group of unreacted nucleic acid strands in the mixture to form covalent bonds, thereby attaching the capture group to the unreacted nucleic acid strands,
(d) removing the unreacted nucleic acid strands from the reaction members using a binding member that binds the capture group, thereby producing a purified library of nucleic acid conjugates.

In some embodiments, each nucleic acid strand comprises a coding sequence that encodes the chemical moiety that is conjugated thereto.

The coding sequence is an identifier which uniquely identifies the chemical moiety or moieties conjugated to that nucleic acid strand. The coding sequence (or coding region) can be any sequence of nucleic acid bases that is uniquely associated with a particular chemical moiety. This allows the identity of the chemical moiety to be determined by sequencing or otherwise 'reading' (i.e., determining the information content) of the coding sequence.

Methods for encoding and decoding chemical moieties, including suitable encoding sequences, are well known in the art (see [1] to [9]).

In some embodiments, a method of making a nucleic acid chemical library as described herein further comprises:
(a) providing a purified library of nucleic acid conjugates comprising nucleic acid strands and chemical moieties,
(b) reacting the library with a diverse population of additional chemical moieties to produce reaction members comprising a diverse population of nucleic acid conjugate products, each nucleic acid conjugate product comprising a combination of a chemical moiety and an additional chemical moiety,
(c) contacting the reaction members with a capping molecule that comprises a capture group, such that the capping molecule reacts with the unreacted nucleic acid conjugates to form covalent bonds, thereby attaching the capture group to unreacted nucleic acid conjugates in the reaction members,
(d) removing the unreacted nucleic acid conjugates using a binding member that binds the capture group, thereby producing a purified library of nucleic acid conjugates comprising different combinations of chemical moieties and additional chemical moieties.

Steps (a) to (d) may be repeated to produce purified libraries of nucleic acid conjugates comprising different combinations of chemical moieties and multiple additional chemical moieties.

A method of making a nucleic acid chemical library as described herein may comprise:
providing a plurality of reaction pools, each comprising nucleic acid strands comprising a first reactive group,
contacting each pool with a different chemical moiety capable of reacting with the first reactive group of the nucleic acid strands to form covalent bonds,
reacting the nucleic acid strands in each pool with the chemical moieties to form reaction members comprising nucleic acid conjugates comprising the chemical moieties, and either;
   (i)
      (a) combining the reaction members in the pools to produce a library of nucleic acid conjugates comprising nucleic acid strands linked to different chemical moieties,
      (b) contacting the library with a capping molecule comprising a capture group, wherein the capping molecule reacts with the first reactive groups of unreacted nucleic acid strands to form covalent bonds, and
      (c) removing unreacted nucleic acid strands from the library using a binding member that binds to the capture moiety to produce a purified library of nucleic acid conjugates, said nucleic acid conjugates comprising nucleic acid strands linked to different chemical moieties.
         or
   (ii)
      (a) contacting the reaction members in each pool with a capping molecule comprising a capture group, wherein the capping molecule reacts with the first reactive groups of unreacted nucleic acid strands to form covalent bonds,
      (b) removing unreacted nucleic acid strands using a binding member that binds to the capture moiety to produce purified populations of nucleic acid conjugates in each pool, and
      (c) combining the purified populations to produce a purified library of nucleic acid conjugates, said nucleic acid conjugates comprising nucleic acid strands linked to different chemical moieties.

A reaction pool comprises one or more reactants. A reaction pool may also contain any necessary fluid phase or catalyst for the reaction to occur.

Initially a reaction pool may comprise nucleic acid strands each comprising a first reactive group. One or more further reactants may subsequently be added to the pool before a reaction occurs. For example, a chemical moiety capable of reacting with the first reactive group of the nucleic acid strands to form covalent bonds.

Where there are a plurality of reaction pools, the reactants in each pool may be different.

For example, a plurality of nucleic acid strands (reactants) comprising a first reactive group may be added to each of a plurality of reaction pools. Each reaction pool may then be contacted with a different chemical moiety (reactant) comprising a further reactive group. Following the reaction between reactive groups, each pool will then contain nucleic acid reaction members covalently bonded to a different chemical moiety. As the reaction is unlikely to go to completion, each reaction pool may also contain unreacted reactants, which will reduce the purity of the population of reaction members in the pool.

In order to remove the unreacted reactants, a capping molecule comprising a capture group may be added to the reaction pool. The reaction members in each pool may be contacted with a capping molecule separately. Alternatively, the different reaction pools may be combined before the capping molecule is added. The capping molecule reacts with the first reactive groups of unreacted nucleic acid strands to form covalent bonds. The unreacted, capped reactants may then be removed using a binding member that binds to the capture moiety.

In some embodiments, the method may further comprise the steps of:
e) splitting the purified library into a plurality of pools,
f) contacting each pool with a different additional chemical moiety, the additional chemical moiety being capable of reacting with the chemical moieties of the nucleic acid conjugates in the pool to form covalent bonds,
g) reacting the nucleic acid conjugates in each pool with the additional chemical moiety to form reaction members comprising nucleic acid conjugate products, the nucleic acid conjugate products comprising combinations of a chemical moiety and an additional chemical moiety, each combination being conjugated to a nucleic acid strand, and either;
   (i)
      (h) combining the reaction members in the pools to produce a library of nucleic acid conjugates comprising combinations of chemical moieties and additional chemical moieties, each combination being conjugated to a nucleic acid strand,
      (i) contacting the library with a capping molecule comprising a capture group, wherein the capping molecule reacts with unreacted nucleic acid conjugate reactants to form covalent bonds, and
      (j) removing unreacted nucleic acid conjugate reactants from the library using a binding member that binds to the capture moiety to produce a purified library of nucleic acid conjugate products comprising a diverse population of combinations of chemical moieties and additional chemical moieties, each combination being conjugated to a nucleic acid strand; or
   (ii)
      (h) contacting the reaction members in each pool with a capping molecule comprising a capture group, wherein the capping molecule reacts with unreacted nucleic acid conjugate reactants to form covalent bonds, and
      (i) removing unreacted, capped nucleic acid conjugate reactants from each pool using a binding member that binds to the capture moiety to produce a population of purified nucleic acid conjugate products in each pool, and
      (j) combining the populations of purified nucleic acid conjugate products in the pools to produce a library of nucleic acid conjugate products comprising combinations of chemical moieties and additional chemical moieties, each combination being conjugated to a nucleic acid strand.

Optionally, steps (e) to (j) may be repeated to add one or more additional chemical moieties to produce a purified library comprising a diverse population of combinations of chemical moieties and two or more additional chemical moieties, each combination being conjugated to a nucleic acid strand.

Optionally, any of the methods described herein may further comprise an encoding step, whereby the identity of the chemical moiety is encoded into the nucleic acid conjugate product. Suitable encoding methods are known in the art, see for example, [10] - [9].

A chemical library may be obtained by any one of the methods described herein.

The chemical library may be a library of DNA conjugates, such as a DNA encoded chemical library.

A kit for use in a method of producing a purified population of nucleic acid conjugate reaction products as described above, may comprise;
a capping molecule comprising a capture group,
wherein the capping molecule is suitable for reacting with a nucleic acid or nucleic acid conjugate reactant comprising a first reactive group to form a covalent bond, and
wherein the capture group is suitable for binding to a binding member.

A kit may include one or more other reagents used in the method, such as buffer solutions, washing solutions and other reagents. A kit may include one or more articles and/or reagents for performance of the method, including suitable binding members and/or chemical moieties as described herein

The disclosure also provides for the use of a capping molecule as described herein in the production of a purified population of nucleic acid conjugate reaction products, including nucleic acid chemical library members, as described above,

Various further aspects and embodiments of the present invention will be apparent to those skilled in the art in view of the present disclosure.

Other aspects and embodiments of the invention provide the aspects and embodiments described above with the term "comprising" replaced by the term "consisting of" and the aspects and embodiments described above with the term "comprising" replaced by the term "consisting essentially of".

It is to be understood that the application discloses all combinations of any of the above aspects and embodiments described above with each other, unless the context demands otherwise. Similarly, the application discloses all combinations of the preferred and/or optional features either singly or together with any of the other aspects, unless the context demands otherwise
"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Certain aspects and embodiments of the invention will now be illustrated by way of example and with reference to the figures described below.

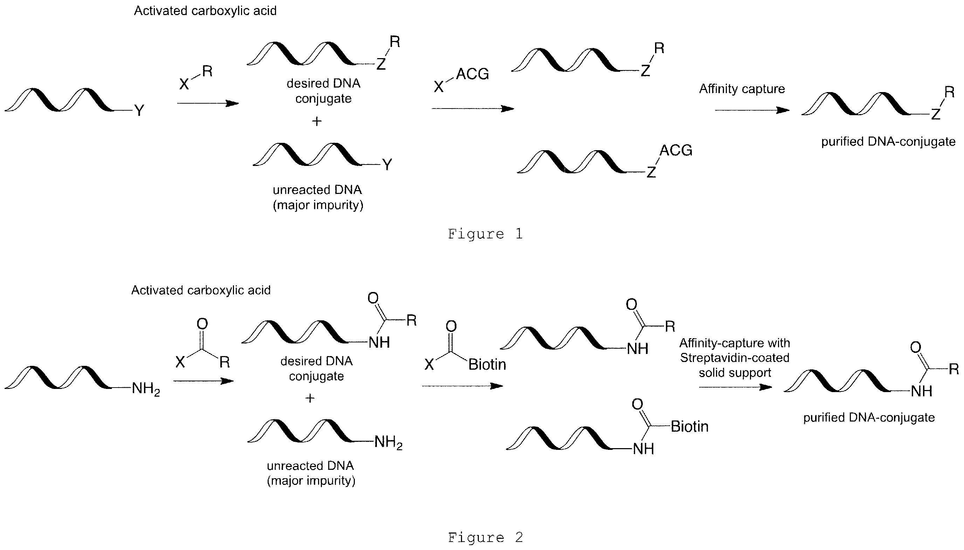

Figure 1 shows a general scheme for the removal of nucleic acid (DNA) or nucleic acid conjugate reactants from nucleic acid conjugate products. In Figure 1 "X" and "Y" indicate reactive groups. "Z" is a linker formed by the reaction of X and Y. R is a building block. "ACG" is an affinity capture group (also called a capture group) which is present on a capping molecule.

In Figure 1, a modified DNA comprising a reactive group (Y), is reacted with the reactive group (X) of building block R. A successful reaction produces the desired DNA conjugate product. However, the reaction often does not go to completion leaving unreacted DNA strands as a major impurity in the reaction products. In order to remove the unreacted DNA, the reaction products are contacted with a capping molecule comprising a capture group(x-ACG). The capping molecule reacts with the reactive group present on the unreacted DNA to link the capture group to the unreacted DNA. The unreacted DNA can then be removed by a binding protein capable of binding to the capture group, while any unreacted building blocks can be removed by a washing step or ethanol precipitation of the DNA conjugates.

Figure 2 shows a reaction scheme in which the capping molecule is an activated carboxylic-acid derivative of biotin. In Figure 2 the DNA reactant is an amino modified DNA and the chemical moiety is a reactive (activated) small molecule carboxylic acid derivative. A successful acylation reaction between the amino modified DNA and an amino-reactive group (X) on the chemical moiety results in the formation of a peptide bond producing the desired DNA conjugate as a reaction product. Unreacted amino modified DNA is a major impurity in the reaction products. The unreacted amino modified DNA is contacted with a capping molecule comprising an activated carboxylic-acid derivative of biotin (for example an NHS-ester or mixed anhydride including Compound A or compound B)). The capping molecule reacts with the unreacted DNA to form a peptide bond. The unreacted DNA can then be removed by affinity capture using a biotin-binding protein (such as streptavidin) coated on a solid support, leaving a purified reaction mixture of the desired DNA conjugate.

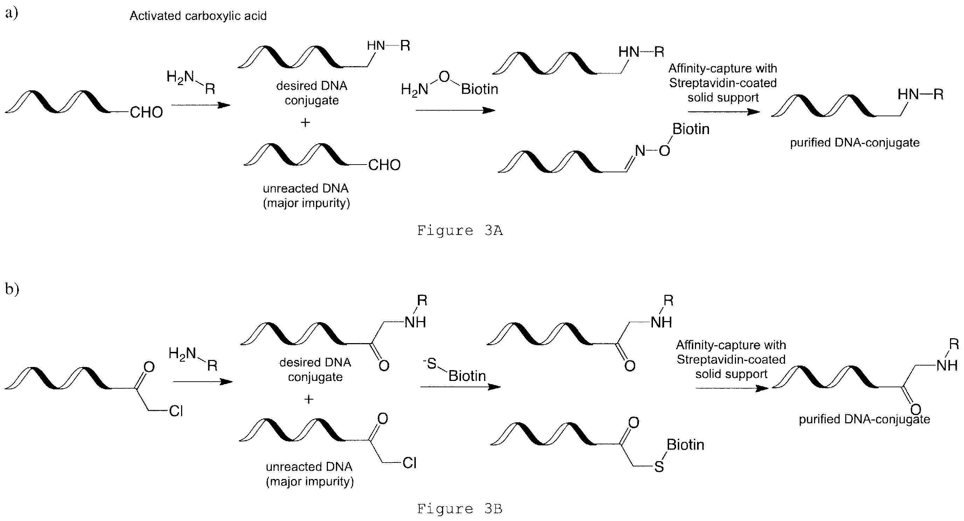

Figures 3a and 3b show reactions which are useful for DNA-encoded chemical library synthesis.

Figure 3a shows a reaction scheme in which the capping molecule is an aldehyde-reactive biotin derivative. The DNA reactant is an aldehyde-modified DNA and the chemical moiety comprises an amine. A reductive amination reaction between aldehyde-modified DNA and the amine group on the chemical moiety results in the formation of a hydrogen bond producing the desired DNA conjugate product. Unreacted aldehyde-modified DNA is a major impurity in the reaction products. Unreacted aldehyde-DNAs can be removed by the reaction with aldehyde-reactive biotin derivatives (e.g. alkoxyamines, 1, 2-mercaptoamines) followed by affinity selection with streptavidin on a solid support, for example streptavidin resin.

Figure 3b shows a reaction scheme in which the capping molecule is a nucleophile containing biotin derivative. The DNA reactant is a chloroacetyl containing DNA and the chemical moiety comprises an amine. A nucleophilic displacement reaction between the chloroacetyl containing DNA and the amine group on the chemical moiety results in the formation of the desired DNA conjugate reaction product. Unreacted chloroacetyl-DNAs reaction products can be removed by the addition of a nucleophile containing biotin derivative (e. g. thiol, phosphorothioate) followed by capture on a solid support, for example streptavidin resin.

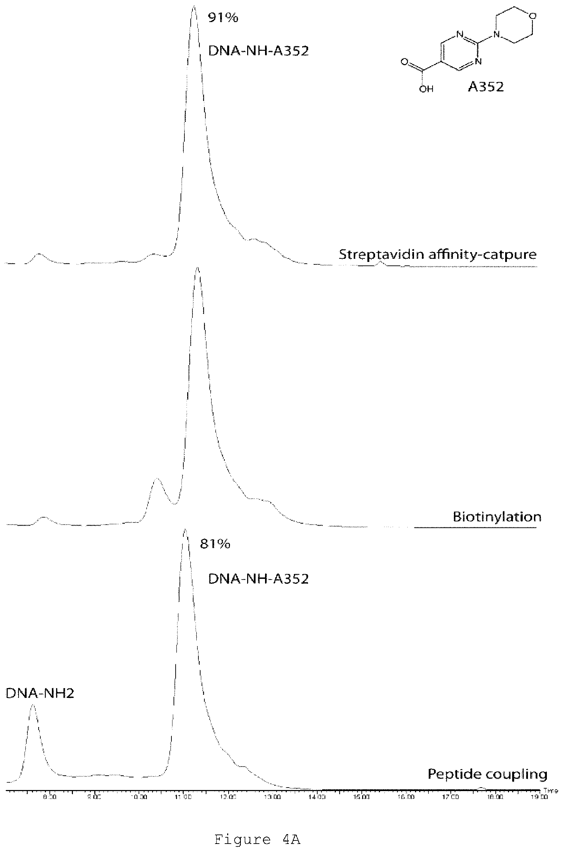

Figure 4a shows the results of example 1 which was carried out according to method 1. Chemical moiety A352 (structure shown in figure 4a) was reacted with amino modified DNA. The bottom chromatogram shows that before purification, both unreacted DNA (DNA-NH2) and reaction product (DNA_NH-A352) are present in the reaction mixture. The middle chromatogram was carried out after contacting the reaction mixture with activated biotin (capping molecule). The top chromatogram was carried out after affinity capture on streptavidin to remove capped unreacted DNA-NH2. The % purity of the reaction product is as indicated.

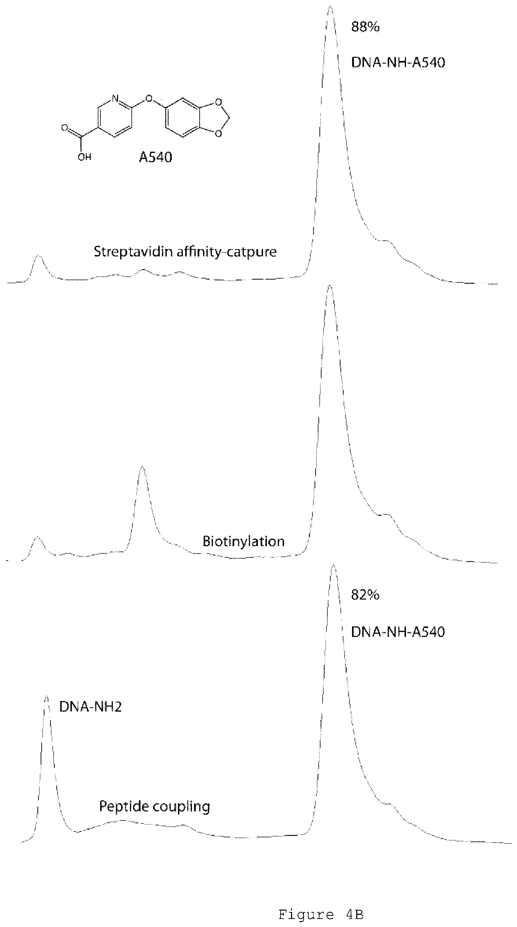

Figure 4b shows the results of example 2 which was carried out according to method 1. Chemical moiety A540 (structure shown in figure 4b) was reacted with amino modified DNA. The bottom chromatogram shows that before purification, both unreacted DNA (DNA-NH2) and reaction product (DNA_NH-A540) are present in the reaction mixture. The middle chromatogram was carried out after contacting the reaction mixture with activated biotin (capping molecule). The top chromatogram was carried out after affinity capture on streptavidin to remove capped unreacted DNA-NH2. The % purity of the reaction product is as indicated.

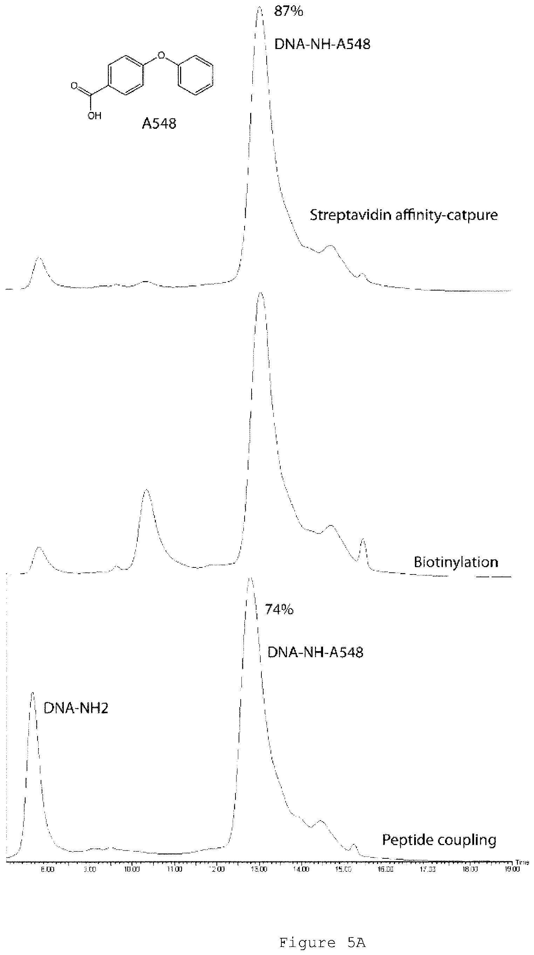

Figure 5a shows the results of example 3 which was carried out according to method 1. Chemical moiety A548 (structure shown in figure 5a) was reacted with amino modified DNA. The bottom chromatogram shows that before purification, both unreacted DNA (DNA-NH2) and reaction product (DNA_NH-A548) are present in the reaction mixture. The middle chromatogram was carried out after contacting the reaction mixture with activated biotin (capping molecule). The top chromatogram was carried out after affinity capture on streptavidin to remove capped unreacted DNA-NH2. The % purity of the reaction product is as indicated.

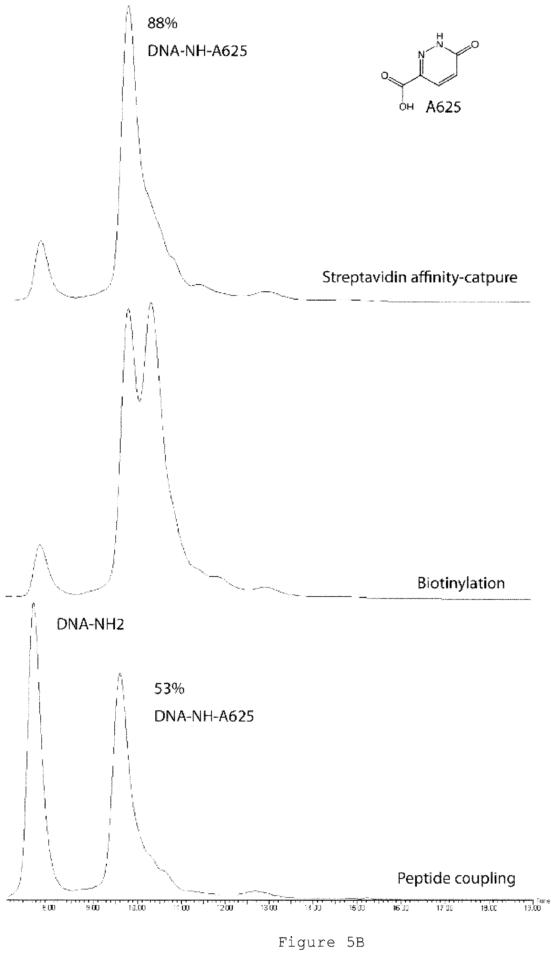

Figure 5b shows the results of example 4 which was carried out according to method 1. Chemical moiety A625 (structure shown in figure 5b) was reacted with amino modified DNA. The bottom chromatogram shows that before purification, both unreacted DNA (DNA-NH2) and reaction product (DNA_NH-A625) are present in the reaction mixture. The middle chromatogram was carried out after contacting the reaction mixture with activated biotin (capping molecule). The top chromatogram was carried out after affinity capture on streptavidin to remove capped unreacted DNA-NH2. The % purity of the reaction product is as indicated.

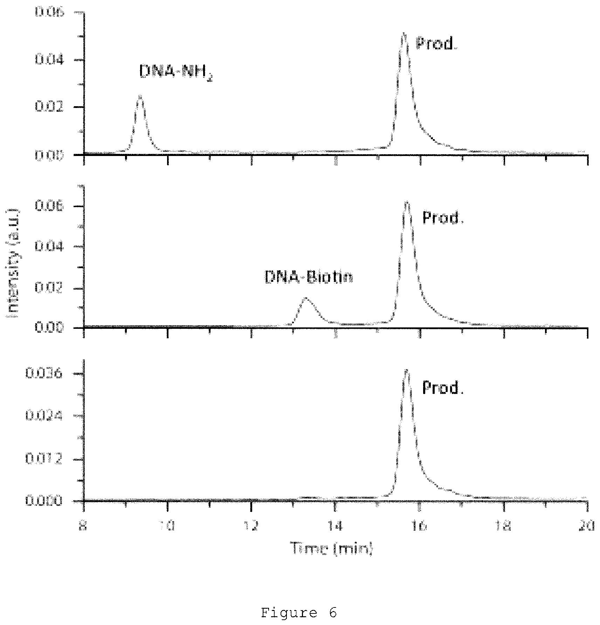

Figure 6 shows the results of example 5 which was carried out according to method 2 and using chemical moiety number 1 from Table 1. Figure 6 (top) shows a HPLC chromatogram of the reaction mixture following a reaction between DNA-NH2 and chemical moiety number 1 from Table 1. A peak is shown for the desired reaction product (prod) and the unreacted DNA reactant; (middle) HPLC chromatogram after capping of unreacted DNAs with a capping molecule (Compound A); (bottom) HPLC chromatogram after affinity capture to remove capped unreacted DNAs. The purity of the reaction product before and after affinity capture is shown in Table 1.

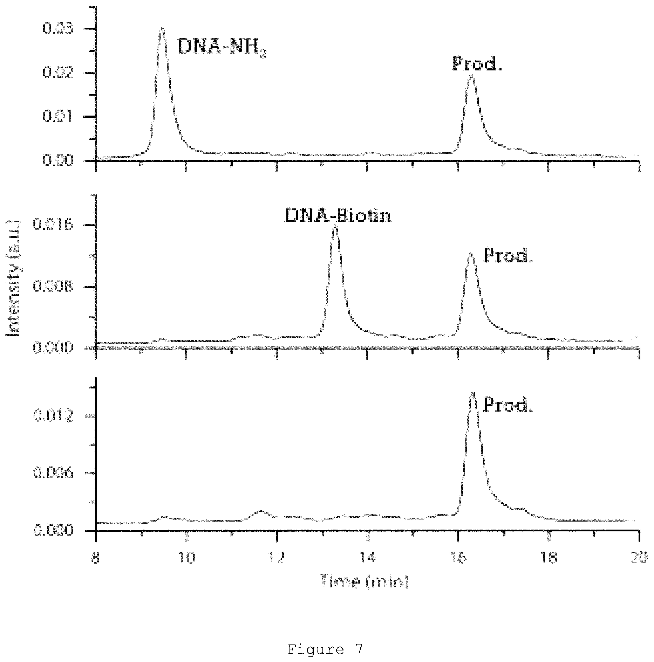

Figure 7 shows the results of example 6 which was carried out according to method 2 and using chemical moiety number 2 from Table 1. Figure 7 (top) shows a HPLC chromatogram of the reaction mixture following a reaction between DNA-NH2 and chemical moiety number 2 from Table 1. A peak is shown for the desired reaction product (prod) and the unreacted DNA reactant; (middle) HPLC chromatogram after capping of unreacted DNAs with a capping molecule (Compound A); (bottom) HPLC chromatogram after affinity capture to remove capped unreacted DNAs. The purity of the reaction product before and after affinity capture is shown in Table 1.

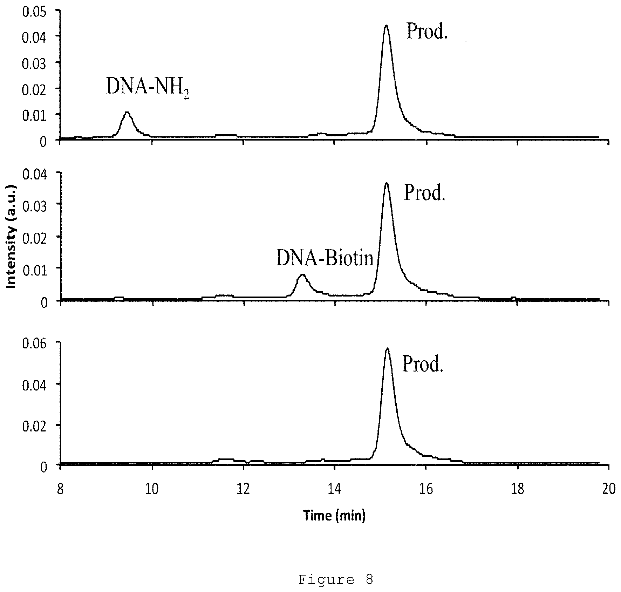

Figure 8 shows the results of example 7 which was carried out according to method 2 and using chemical moiety number 3 from Table 1. Figure 8 (top) shows a HPLC chromatogram of the reaction mixture following a reaction between DNA-NH2 and chemical moiety number 3 from Table 1. A peak is shown for the desired reaction product (prod) and the unreacted DNA reactant; (middle) HPLC chromatogram after capping of unreacted DNAs with a capping molecule (Compound A); (bottom) HPLC chromatogram after affinity capture to remove capped unreacted DNAs. The purity of the reaction product before and after affinity capture is shown in Table 1.

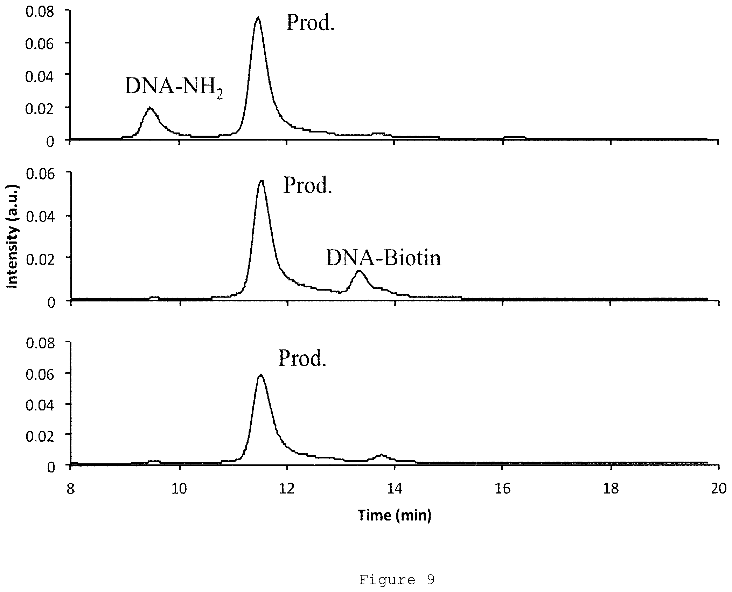

Figure 9 shows the results of example 8 which was carried out according to method 2 and using chemical moiety number 4 from Table 1. Figure 9 (top) shows a HPLC chromatogram of the reaction mixture following a reaction between DNA-NH2 and chemical moiety number 4 from Table 1. A peak is shown for the desired reaction product (prod) and the unreacted DNA reactant; (middle) HPLC chromatogram after capping of unreacted DNAs with a capping molecule (Compound A); (bottom) HPLC chromatogram after affinity capture to remove capped unreacted DNAs. The purity of the reaction product before and after affinity capture is shown in Table 1.

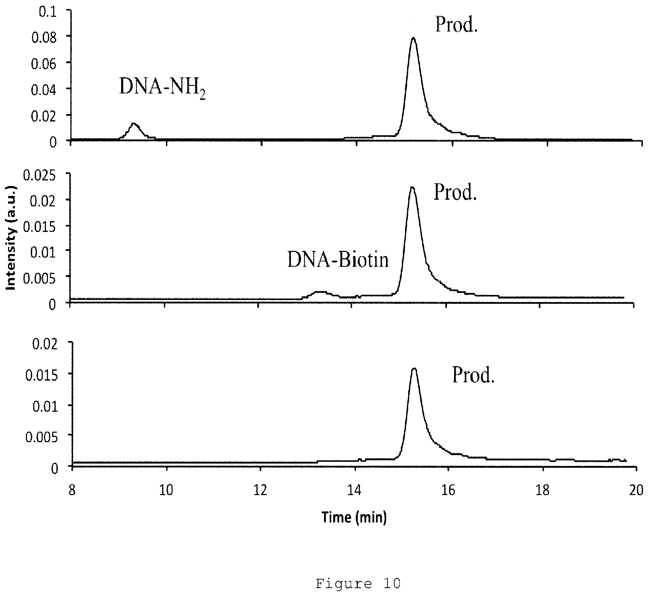

Figure 10 shows the results of example 9 which was carried out according to method 2. The chemical moiety was number 5 from Table 1. Figure 10 (top) shows a HPLC chromatogram of the reaction mixture following a reaction between DNA-NH2 and chemical moiety number 5 from Table 1. A peak is shown for the desired reaction product (prod) and the unreacted DNA reactant; (middle) HPLC chromatogram after capping of unreacted DNAs with a capping molecule (Compound A); (bottom) HPLC chromatogram after affinity capture to remove capped unreacted DNAs. The purity of the reaction product before and after affinity capture is shown in Table 1.

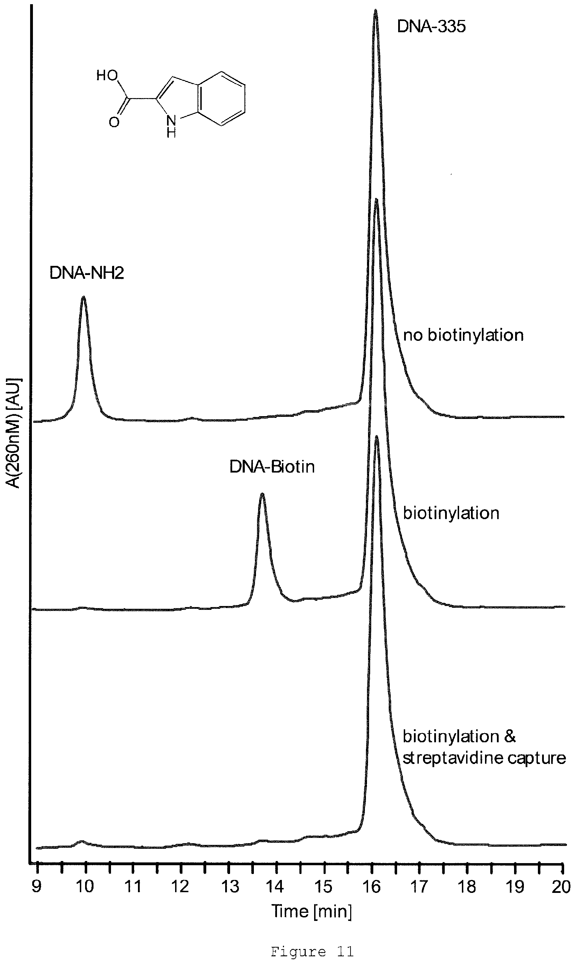

Figure 11 shows the results of example 10, which was carried out according to method 3 and using chemical moiety A335 (structure shown in figure). Figure 11 (top) shows a HPLC chromatogram of the reaction mixture following a reaction between DNA-NH2 and chemical moiety number A335. A peak is shown for the desired reaction product (prod) and the unreacted DNA reactant; (middle) HPLC chromatogram after capping of unreacted DNAs with a capping molecule (Compound B); (bottom) HPLC chromatogram after affinity capture to remove capped unreacted DNAs.

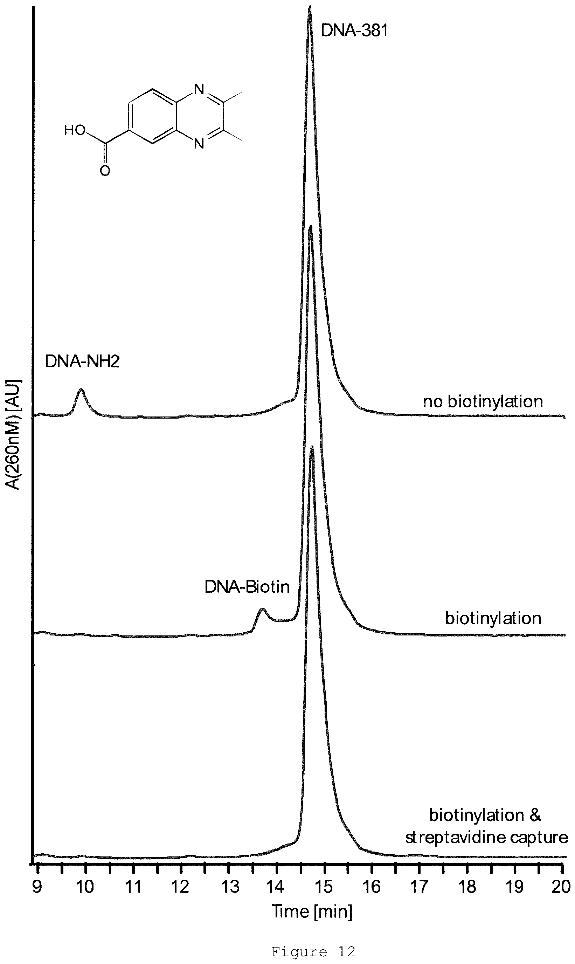

Figure 12 shows the results of example 11, which was carried out according to method 3 and using chemical moiety A381 (structure shown in figure). Figure 12 (top) shows a HPLC chromatogram of the reaction mixture following a reaction between DNA-NH2 and chemical moiety number A381. A peak is shown for the desired reaction product (prod) and the unreacted DNA reactant; (middle) HPLC chromatogram after capping of unreacted DNAs with a capping molecule (Compound B); (bottom) HPLC chromatogram after affinity capture to remove capped unreacted DNAs.

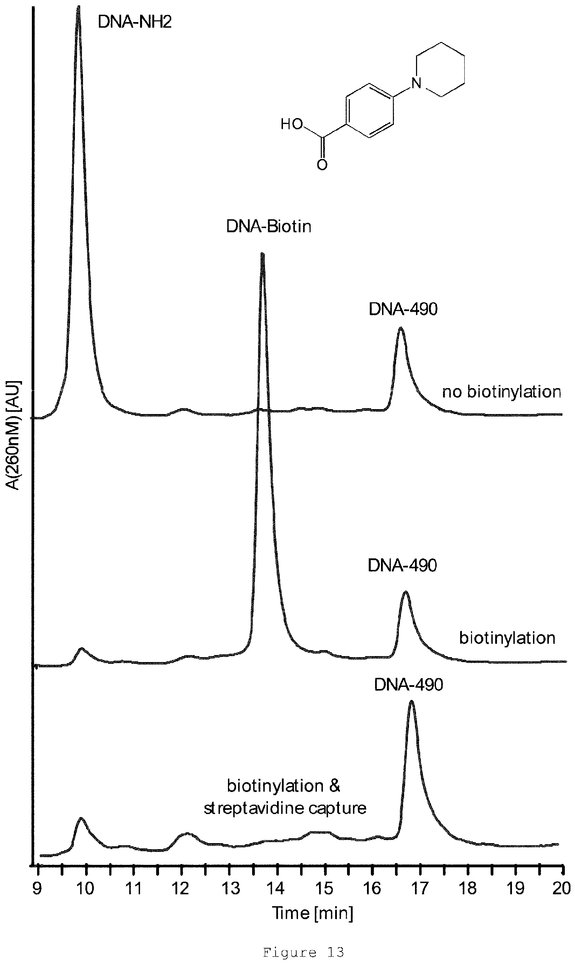

Figure 13 shows the results of example 12, which was carried out according to method 3 and using chemical moiety A490 (structure shown in figure). Figure 13 (top) shows a HPLC chromatogram of the reaction mixture following a reaction between DNA-NH2 and chemical moiety number A490. A peak is shown for the desired reaction product (prod) and the unreacted DNA reactant; (middle) HPLC chromatogram after capping of unreacted DNAs with a capping molecule (Compound B); (bottom) HPLC chromatogram after affinity capture to remove capped unreacted DNAs.

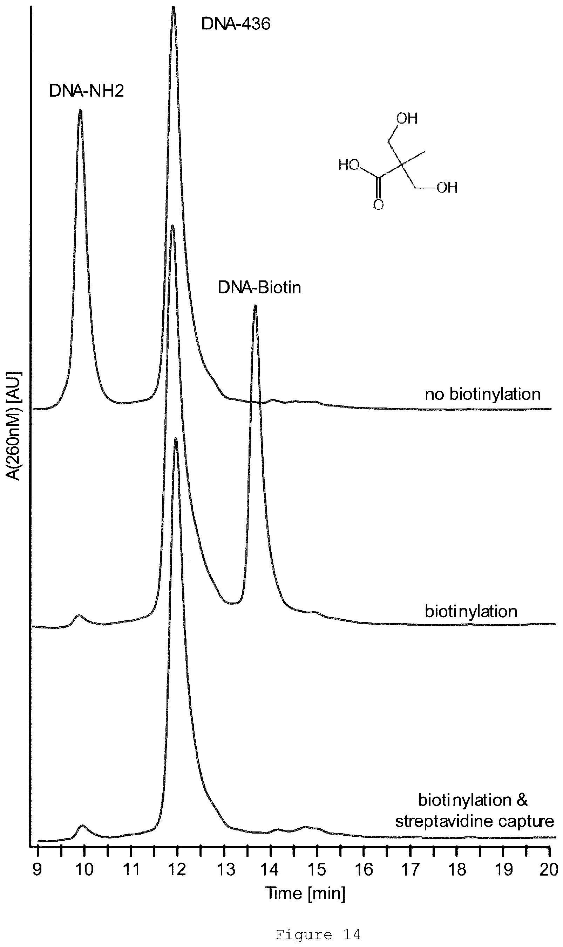

Figure 14 shows the results of example 13, which was carried out according to method 3 and using chemical moiety A436 (structure shown in figure). Figure 14 (top) shows a HPLC chromatogram of the reaction mixture following a reaction between DNA-NH2 and chemical moiety number A436. A peak is shown for the desired reaction product (prod) and the unreacted DNA reactant; (middle) HPLC chromatogram after capping of unreacted DNAs with a capping molecule (Compound B); (bottom) HPLC chromatogram after affinity capture to remove capped unreacted DNAs.

### Experiments

### Materials and Methods

The desired DNA conjugates may be generated by the condensation of an amino modified DNA with an activated carboxylic acid to form an amide bond. As a general method, DEAE sepharose (Whatman, GE Healthcare) is washed with 10 mM aq. AcOH (2 x) and water (2 x). DNA-C₁₂NH₂ (1 nmol) is immobilized on the anion exchange resin by incubation of an aqueous solution of the DNA for 5 min, followed by washing with 10 mM aq. AcOH (2 x) and DMSO (2 x). A solution of an acid (50 mM), EDC (50 mM) and HOAt (5 mM) in DMSO (500 µL) is preincubated for 5 min and added to the immobilized DNA. The reaction mixture is gently shaken for 2 h. The resin is rinsed with DMSO (2 x). The coupling and washing steps are repeated (2 x). Then, the resin is washed with DMSO (2 x).

Capped nucleic acid conjugates were generated according to the following exemplary methods:

### Method 1

A solution of biotin (free carboxylic acid) (50 mM), 1-Ethyl-3-(3-dimetylaminopropyl) carbodiimide (EDC, 50 mM) and 1-Hydroxy-7-azatriazole (HOAt, 5mM) in DMSO (500 uL) is preincubated for 5 min and added to the immobilized DNA. The reaction mixture is shaken overnight (16h). The resin is washed with DMSO (2 x) and 10 mM aq. AcOH (2 x). The DNA is eluted with AcOH buffer (3.0 M, pH 4.75) and precipitated with EtOH. Affinity capture of capped DNAs is performed as described below.

Method 1 was used in examples 1-4 (Figures 4a-5b)

### Method 2

### Preparation of the capping molecule Compound A (biotin isobutyloxy anhydride) and capping of unreacted amine-DNA conjugates

Diisopropylethylamine (DIPEA, 1.5mmol) is added to a solution of carboxylic acid biotin (1mmol) in dry DMSO (8ml) under argon and stirred at 0°C followed by the dropwise addition of isobutyl chloroformate (0.8 mmol). The reaction mixture is stirred for Ih forming Compound A (biotin isobutyloxy anhydride) Freshly prepared Compound A is immediately added to the immobilized DNA (see above) and shaken overnight (16h).

The resin is washed with DMSO (2 x) and 10 mM aq. AcOH (2 x). The DNA is eluted with AcOH buffer (3.0 M, pH 4.75) and precipitated with EtOH. Affinity capture of capped DNAs is performed as described below.

Method 2 was used in examples 5-9 (Figures 6-10).

### Method 3

A solution of biotin N-hydroxysuccinimidyl ester (compound B; 200 mM) and 4-dimethylaminoaniline (20 mM) in DMSO (200 uL) was added to the immobilized DNA (1-2 nmol). The reaction mixture is shaken for 4 h at 37 °C. The resin is washed with DMSO (2 x) and 10 mM aq. AcOH (2 x). The DNA is eluted with AcOH buffer (3.0 M, pH 4.75) and precipitated with EtOH. Affinity capture of capped DNAs is performed as described below.

Method 3 was used in examples 10-13.

Affinity capture:
Streptavidin Sepharose High Performance (Amersham Biosciences) is washed with H₂O (2 x) and PBS (2 x). DNA solution (1.8 nmol) in PBS is added on the streptavidin resin and incubated for 15 min, then centrifuged (2 x). DNA conjugates are analysed directly by HPLC or further processed by EtOH precipitation.

Table 1 shows the chemical structures of chemical moieties 1-5 used in examples 5-9 (Figures 6-10). The purity of the reaction product before and after purification is also indicated.

**Table 1: Affinity capture efficiencies (Method 2):**

| | Chemical structures | Product fraction Before affinity capture | Product fraction after affinity capture |
|---|---|---|---|
| 1 | 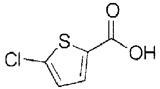 | 77% | 100% |
| 2 | 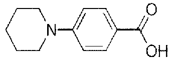 | 45% | 80% |
| 3 | 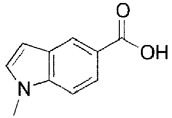 | 81% | 95% |
| 4 | 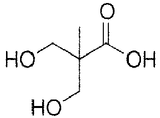 | 79% | 90% |
| 5 | 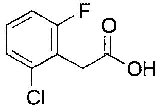 | 90% | 100% |

### Examples

1. Example 1 was carried out according to method 1. Chemical moiety A352 (structure shown in Figure 4a) was reacted with amino modified DNA. Unreacted amino-modified DNA reactants were removed using biotin as a capping molecule followed by affinity capture on streptavidin. The results, including the % purity of the reaction product are shown in Figure 4a.
2. Example 2 was carried out according to method 1. Chemical moiety A540 (structure shown in Figure 4b) was reacted with amino modified DNA. Unreacted amino-modified DNA reactants were removed using biotin as a capping molecule followed by affinity capture on streptavidin. The results, including the % purity of the reaction product are shown in Figure 4b.
3. Example 3 was carried out according to method 1. Chemical moiety A548 (structure shown in Figure 5a) was reacted with amino modified DNA. Unreacted amino-modified DNA reactants were removed using biotin as a capping molecule followed by affinity capture on streptavidin. The results, including the % purity of the reaction product are shown in Figure 5a.
4. Example 4 was carried out according to method 1. Chemical moiety A625 (structure shown in Figure 5b) was reacted with amino modified DNA. Unreacted amino-modified DNA reactants were removed using biotin as a capping molecule followed by affinity capture on streptavidin. The results, including the % purity of the reaction product are shown in Figure 5b.

Examples 5-9 were carried out according to method 2. In examples 5-9, respectively, chemical moieties 1-5 from Table 1 were reacted with amino modified DNA. Unreacted amino-modified DNA was capped with a capping molecule (Compound A) and removed by affinity capture. The purity of the reaction product before and after affinity capture is shown in Table 1.

Example 10 was carried out according to method 3. Chemical moiety A335 (structure shown in Figure 11) was reacted with amino modified DNA. Unreacted amino-modified DNA reactants were removed using biotin as a capping molecule followed by affinity capture on streptavidin. The results of the reaction product and subsequent streptavidin purification are shown in Figure 11.

Examples 11-13 were carried out according to method 3. In examples 11-13, respectively, chemical moieties shown in Figures 12-14 (A381, A490, and A436) were reacted with amino modified DNA. Unreacted amino-modified DNA was capped with a capping molecule (Compound B) and removed by streptavidin affinity capture. The results of the reaction product and subsequent streptavidin purification are shown in Figures 12-14.

### References

1 Mannocci, L. et al. PNAS USA 105(46):17670-17675
2 Brenner, S. and Lerner, R.A.. PNAS USA 89 (1992), 5381-5383
3 Nielsen, J., et al., J. Am. Chem. Soc. 115 (1993)
4 Needels et al., M.C., PNAS USA 90 (1993), 10700-10704
5 Gartner, Z.J., et al., Science 305 (2004), 1601-1605
6 Melkko, S., et al., Nat. Biotechnol. 22 568-574 (2004)
7 Sprinz, K.I., et al., Bioorg. Med. Chem. Lett. 15 (2005), pp. 3908-3911
8 Leimbacher et al Chemistry. 2012 Jun 18;18(25):7729-37
9 Clark et al Nat Chem Biol. 2009 Sep;5(9):647-54
10 Wuts, P.G.L and Greene, T.W. Greene's Protective Groups in Organic Synthesis, Fourth Edition (2006).

## Claims

1. A method of producing a purified population of nucleic acid conjugate reaction products comprising;
providing reaction members that comprise nucleic acid conjugate reaction products and nucleic acid or nucleic acid conjugate reactants, wherein said reactants comprise a first reactive group,
contacting the reaction members with a capping molecule comprising a capture group, wherein the capping molecule reacts with the first reactive group to form a covalent bond, thereby attaching the capture group to nucleic acid or nucleic acid conjugate reactants in reaction members,
removing the nucleic acid or nucleic acid conjugate reactants from the reaction members using a binding member that binds the capture group,
thereby producing a purified population of nucleic acid conjugate products.

2. A method according to claim 1 wherein the reaction members are provided by a method comprising;
reacting a population of nucleic acid or nucleic acid conjugate reactants having a first reactive group with a population of chemical moieties that react with the first reactive group to produce reaction members comprising nucleic acid conjugate products that contain the chemical moiety and unreacted nucleic acid or nucleic acid conjugate reactants.

3. A method according to claim 1 or claim 2 wherein the reaction members comprise nucleic acid strands having a first reactive group, optionally wherein the first reactive group is at a terminal of the nucleic acid strands.

4. A method according to claim 1 or claim 2 wherein the reaction members comprise nucleic acid conjugate reactants having a first reactive group, optionally wherein the nucleic acid conjugate reactants comprise a nucleic acid strand conjugated to at least one chemical moiety and wherein the conjugated chemical moiety comprises the first reactive group.

5. A method according to claim 4 wherein the nucleic acid conjugate reactants comprise a nucleic acid strand linked to n chemical moieties, where n is 1 to 10, and the nucleic acid conjugate products comprise a nucleic acid molecule linked to n+1 chemical moieties.

6. A method according to any one of claims 1 to 5 wherein the nucleic acid or nucleic acid-conjugate reactants are removed by contacting the reaction members with the binding member and separating from the reaction products nucleic acid or nucleic acid conjugate reactants that bind to the binding member.

7. A method according to any one of claims 1 to 6 wherein the binding member is immobilised on a solid support.

8. A method according to any one of claims 1 to 7 wherein the capture group comprises biotin or a biotin derivative.

9. A method according to claim 8 wherein the biotin derivative is desthiobiotin or iminobiotin.

10. A method according to any one of claims 8 to 9 wherein the binding member comprises a biotin-binding protein.

11. A method according to claim 10 wherein the biotin-binding protein is streptavidin or avidin.

12. A method according to any one of claims 2 to 8 wherein the chemical moiety comprises a second reactive group that reacts with the first reactive group to form a covalent bond and the capture molecule comprises a third reactive group that reacts with the first reactive group to form a covalent bond.

13. A method according to claim 12, wherein:
(a) the first reactive group is an amine and the second and third reactive groups are activated carboxylic acid-derivatives, or vice versa;
(b) the first reactive group is an aldehyde and one of the second and third reactive groups is an alkoxyamine or 1, 2-mercaptoamine and the other is a primary amine; or
(c) the first reactive group is a chloroacetyl group and one of the second and third reactive groups is a nucleophilic group, such as thiol or phosphorothioate, and the other is a primary amine.

14. A method according to any one of claims 1 to 13 further comprising
(a) reacting the purified population of nucleic acid conjugates with a population of additional chemical moieties reactive with the chemical moiety of the nucleic acid conjugate, to produce reaction products comprising nucleic acid conjugate products comprising the additional chemical moiety and nucleic acid conjugate reactants,
(b) contacting the mixture with a capping molecule that comprises a capture group, wherein the capping group reacts with the chemical moiety of the nucleic acid conjugate reactants in the reaction products to form covalent bonds, thereby attaching the capture group to nucleic acid conjugate reactants, and,
(c) removing the unreacted nucleic acid conjugate reactants from the reaction products using a binding member that binds the capture group,
thereby producing a purified population of nucleic acid conjugate products comprising the additional chemical moiety,
optionally wherein the method comprises repeating steps (a) to (c) one or more times to produce a purified population of nucleic acid conjugate products comprising two or more additional chemical moieties.

15. A method of making a nucleic acid chemical library comprising:
(a) providing a population of nucleic acid strands, each strand comprising a first reactive group,
(b) reacting the nucleic acid strands with a diverse population of chemical moieties capable of reacting with the first reactive group to produce reaction products comprising a diverse population of nucleic acid conjugates that comprise nucleic acid strands conjugated to chemical moieties,
(c) contacting the reaction products with a capping molecule that comprises a capture group, wherein the capping molecule reacts with the first reactive group of unreacted nucleic acid strands in the mixture to form covalent bonds, thereby attaching the capture group to the unreacted nucleic acid strands,
(d) removing the unreacted nucleic acid strands from the reaction products using a binding member that binds the capture group, thereby producing a purified library of nucleic acid conjugates.

16. A method according to claim 15 wherein each nucleic acid strand comprises a coding sequence that encodes the chemical moiety that is conjugated thereto.

17. A method according to claim 15 or 16 further comprising:
(a) providing a purified library of nucleic acid conjugates comprising nucleic acid strands and chemical moieties,
(b) reacting the library with a diverse population of additional chemical moieties to produce reaction products comprising a diverse population of nucleic acid conjugate products, each nucleic acid conjugate product comprising a combination of a chemical moiety and an additional chemical moiety,
(c) contacting the reaction products with a capping molecule that comprises a capture group, such that the capping molecule reacts with the unreacted nucleic acid conjugates to form covalent bonds, thereby attaching the capture group to unreacted nucleic acid conjugates in the mixture,
(d) removing the unreacted nucleic acid conjugates using a binding member that binds the capture group,
thereby producing a purified library of nucleic acid conjugates comprising different combinations of chemical moieties and additional chemical moieties,
optionally wherein the method comprises repeating steps (a) to (d) and further comprises:
e) splitting the purified library into a plurality of pools,
f) contacting each pool with a different additional chemical moiety, the additional chemical moiety being capable of reacting with the chemical moieties of the nucleic acid conjugates in the pool to form covalent bonds,
g) reacting the nucleic acid conjugates in each pool with the additional chemical moiety to form reaction products comprising nucleic acid conjugate products, the nucleic acid conjugate products comprising combinations of a chemical moiety and an additional chemical moiety, each combination being conjugated to a nucleic acid strand, and either;
(i)
(h) combining the reaction products in the pools to produce a library of nucleic acid conjugates comprising combinations of chemical moieties and additional chemical moieties, each combination being conjugated to a nucleic acid strand,
(i) contacting the library with a capping molecule comprising a capture group, wherein the capping molecule reacts with unreacted nucleic acid conjugate reactants to form covalent bonds, and
(j) removing unreacted nucleic acid conjugate reactants from the library using a binding member that binds to the capture moiety to produce a purified library of nucleic acid conjugate products comprising a diverse population of combinations of chemical moieties and additional chemical moieties, each combination being conjugated to a nucleic acid strand; or
(ii)
(h) contacting the reaction products in each pool with a capping molecule comprising a capture group, wherein the capping molecule reacts with unreacted nucleic acid conjugate reactants to form covalent bonds, and
(i) removing unreacted nucleic acid conjugate reactants from each pool using a binding member that binds to the capture moiety to produce a population of purified nucleic acid conjugate products in each pool, and
(j) combining the populations of purified nucleic acid conjugate products in the pools to produce a library of nucleic acid conjugate products comprising combinations of chemical moieties and additional chemical moieties, each combination being conjugated to a nucleic acid strand, and
optionally wherein the method comprises repeating steps (e) to (j) to add one or more additional chemical moieties to produce a purified library comprising a diverse population of combinations of chemical moieties and two or more additional chemical moieties, each combination being conjugated to a nucleic acid strand.

18. A method of making a nucleic acid chemical library comprising
providing a plurality of reaction pools, each comprising nucleic acid strands comprising a first reactive group,
contacting each pool with a different chemical moiety capable of reacting with the first reactive group of the nucleic acid strands to form covalent bonds,
reacting the nucleic acid strands in each pool with the chemical moieties to form reaction products comprising nucleic acid conjugates comprising the chemical moieties, and either;
(i)
(a) combining the reaction products in the pools to produce a library of nucleic acid conjugates comprising nucleic acid strands linked to different chemical moieties,
(b) contacting the library with a capping molecule comprising a capture group, wherein the capping molecule reacts with the first reactive groups of unreacted nucleic acid strands to form covalent bonds, and
(c) removing unreacted nucleic acid strands from the library using a binding member that binds to the capture moiety to produce a purified library of nucleic acid conjugates, said nucleic acid conjugates comprising nucleic acid strands linked to different chemical moieties; or
(ii)
(a) contacting the reaction products in each pool with a capping molecule comprising a capture group, wherein the capping molecule reacts with the first reactive groups of unreacted nucleic acid strands to form covalent bonds,
(b) removing unreacted nucleic acid strands using a binding member that binds to the capture moiety to produce purified populations of nucleic acid conjugates in each pool, and
(c) combining the purified populations to produce a purified library of nucleic acid conjugates, said nucleic acid conjugates comprising nucleic acid strands linked to different chemical moieties.

## Patentansprüche

1. Verfahren zur Herstellung einer gereinigten Population von Nucleinsäurekonjugatreaktionsprodukten, das Folgendes umfasst:
das Bereitstellen von Reaktionselementen, die Nucleinsäurekonjugatreaktionsprodukte und Nucleinsäure- oder Nucleinsäurekonjugatreaktanten umfassen, wobei die Reaktanten eine erste reaktive Gruppe umfassen,
das Kontaktieren der Reaktionselemente mit einem Verkappungsmolekül, das eine Fanggruppe umfasst, wobei das Verkappungsmolekül mit der ersten reaktiven Gruppe reagiert, um eine kovalente Bindung zu bilden, wodurch die Fanggruppe an Nucleinsäure- oder Nucleinsäurekonjugatreaktanten in Reaktionselementen gebunden wird,
das Entfernen der Nucleinsäure- oder Nucleinsäurekonjugatreaktanten aus den Reaktionselementen unter Verwendung eines Bindungselements, das die Fanggruppe bindet,
wodurch eine gereinigte Population von Nucleinsäurekonjugatprodukten erzeugt wird.

2. Verfahren nach Anspruch 1, wobei die Reaktionselemente durch ein Verfahren bereitgestellt werden, das Folgendes umfasst:
das Umsetzen einer Population von Nucleinsäure- oder Nucleinsäurekonjugatreaktanten mit einer ersten reaktiven Gruppe mit einer Population chemischer Gruppierungen, die mit der ersten reaktiven Gruppe reagieren, zur Herstellung von Reaktionselementen, die Nucleinsäurekonjugatprodukte umfassen, die die chemische Gruppierung und nicht umgesetzte Nucleinsäure- oder Nucleinsäurekonjugatreaktanten enthalten.

3. Verfahren nach Anspruch 1 oder 2, wobei die Reaktionselemente Nucleinsäurestränge mit einer ersten reaktiven Gruppe umfassen, wobei die erste reaktive Gruppe gegebenenfalls endständig an den Nucleinsäuresträngen vorliegt.

4. Verfahren nach Anspruch 1 oder 2, wobei die Reaktionselemente Nucleinsäurekonjugatreaktanten mit einer ersten reaktiven Gruppe umfassen, wobei die Nucleinsäurekonjugatreaktanten gegebenenfalls einen Nucleinsäurestrang umfassen, der an zumindest eine chemische Gruppierung konjugiert ist, und wobei die konjugierte chemische Gruppierung die erste reaktive Gruppe umfasst.

5. Verfahren nach Anspruch 4, wobei die Nucleinsäurekonjugatreaktanten einen Nucleinsäurestrang umfassen, der an n chemische Gruppierungen gebunden ist, wobei n = 1 bis 10 ist, und die Nucleinsäurekonjugatprodukte ein Nucleinsäuremolekül umfassen, das an n+1 chemische Gruppierungen gebunden ist.

6. Verfahren nach einem der Ansprüche 1 bis 5, wobei die Nucleinsäure- oder Nucleinsäurekonjugatreaktanten durch Kontaktieren der Reaktionselemente mit dem Bindungselement und Abtrennen von Nucleinsäure- oder Nucleinsäurekonjugatreaktanten, die an das Bindungselement binden, von den Reaktionsprodukten entfernt werden.

7. Verfahren nach einem der Ansprüche 1 bis 6, wobei das Bindungselement auf einem festen Träger immobilisiert ist.

8. Verfahren nach einem der Ansprüche 1 bis 7, wobei die Fanggruppe Biotin oder ein Biotinderivat umfasst.

9. Verfahren nach Anspruch 8, wobei das Biotinderivat Desthiobiotin oder Iminobiotin ist.

10. Verfahren nach einem der Ansprüche 8 bis 9, wobei das Bindungselement ein Biotin-Bindungsprotein umfasst.

11. Verfahren nach Anspruch 10, wobei das Biotin-Bindungsprotein Streptavidin oder Avidin ist.

12. Verfahren nach einem der Ansprüche 2 bis 8, wobei die chemische Gruppierung eine zweite reaktive Gruppe umfasst, die mit der ersten reaktiven Gruppe reagiert, um eine kovalente Bindung zu bilden, und das Fangmolekül eine dritte reaktive Gruppe umfasst, die mit der ersten reaktiven Gruppe reagiert, um eine kovalente Bindung zu bilden.

13. Verfahren nach Anspruch 12, wobei:
(a) die erste reaktive Gruppe ein Amin ist und die zweite und die dritte reaktive Gruppe aktivierte Carbonsäurederivate sind oder umgekehrt;
(b) die erste reaktive Gruppe ein Aldehyd ist und eine der zweiten und dritten reaktiven Gruppe ein Alkoxyamin oder 1,2-Mercaptoamin und die andere ein primäres Amin ist; oder
(c) die erste reaktive Gruppe eine Chloracetylgruppe ist und eine der zweiten und dritten reaktiven Gruppe eine nucleophile Gruppe, wie z.B. Thiol oder Thiophosphat, und die andere ein primäres Amin ist.

14. Verfahren nach einem der Ansprüche 1 bis 13, das des Weiteren Folgendes umfasst:
(a) das Umsetzen der gereinigten Population von Nucleinsäurekonjugaten mit einer Population zusätzlicher chemischer Gruppierungen, die mit der chemischen Gruppierung des Nucleinsäurekonjugats reaktiv sind, zur Herstellung von Reaktionsprodukten, die Nucleinsäurekonjugatprodukte umfassen, die die zusätzliche chemische Gruppierung und Nucleinsäurekonjugatreaktanten umfassen,
(b) das Kontaktieren des Gemischs mit einem Verkappungsmolekül, das eine Fanggruppe umfasst, wobei die Verkappungsgruppe mit der chemischen Gruppierung der Nucleinsäurekonjugatreaktanten in den Reaktionsprodukten reagiert, um kovalente Bindungen zu bilden, wodurch die Fanggruppe an die Nucleinsäurekonjugatreaktanten gebunden wird, und
(c) das Entfernen der nicht umgesetzten Nucleinsäurekonjugatreaktanten aus den Reaktionsprodukten unter Verwendung eines Bindungselements, das die Fanggruppe bindet,
wodurch eine gereinigte Population von Nucleinsäurekonjugatprodukten hergestellt wird, die die zusätzliche chemische Gruppierung umfassen,
wobei das Verfahren gegebenenfalls das ein- oder mehrmalige Wiederholen der Schritte (a) bis (c) umfasst, um eine gereinigte Population von Nucleinsäurekonjugatprodukten herzustellen, die zwei oder mehr zusätzliche chemische Gruppierungen umfassen.

15. Verfahren zur Herstellung einer chemischen Nucleinsäurebibliothek, das Folgendes umfasst:
(a) das Bereitstellen einer Population von Nucleinsäuresträngen, wobei jeder Strang eine erste reaktive Gruppe umfasst,
(b) das Umsetzen der Nucleinsäurestränge mit einer vielfältigen Population von chemischen Gruppierungen, die in der Lage sind, mit der ersten reaktiven Gruppe zu reagieren, um Reaktionsprodukte zu erzeugen, die eine vielfältige Population von Nucleinsäurekonjugaten umfassen, die Nucleinsäurestränge konjugiert an chemische Gruppierungen umfassen,
(c) das Kontaktieren der Reaktionsprodukte mit einem Verkappungsmolekül, das eine Fanggruppe umfasst, wobei das Verkappungsmolekül mit der ersten reaktiven Gruppe von nicht umgesetzten Nucleinsäuresträngen in dem Gemisch reagiert, um kovalente Bindungen zu bilden, wodurch die Fanggruppe an die nicht umgesetzten Nucleinsäurestränge gebunden wird,
(d) das Entfernen der nicht umgesetzten Nucleinsäurestränge aus den Reaktionsprodukten unter Verwendung eines Bindungselements, das die Fanggruppe bindet, wodurch eine gereinigte Bibliothek von Nucleinsäurekonjugaten hergestellt wird.

16. Verfahren nach Anspruch 15, wobei jeder Nucleinsäurestrang eine Kodierungssequenz umfasst, die für die chemische Gruppierung kodiert, die an diesen konjugiert ist.

17. Verfahren nach Anspruch 15 oder 16, das des Weiteren Folgendes umfasst:
(a) das Bereitstellen einer gereinigten Bibliothek von Nucleinsäurekonjugaten, die Nucleinsäurestränge und chemische Gruppierungen umfassen,
(b) das Umsetzen der Bibliothek mit einer vielfältigen Population von weiteren chemischen Gruppierungen zur Herstellung von Reaktionsprodukten, die eine vielfältige Population von Nucleinsäurekonjugatprodukten umfassen, wobei jedes Nucleinsäurekonjugatprodukt eine Kombination von einer chemischen Gruppierung und einer weiteren chemischen Gruppierung umfasst,
(c) das Kontaktieren der Reaktionsprodukte mit einem Verkappungsmolekül, das eine Fanggruppe umfasst, so dass das Verkappungsmolekül mit den nicht umgesetzten Nucleinsäurekonjugaten reagiert, um kovalente Bindungen zu bilden, wodurch die Fanggruppe an die nicht umgesetzten Nucleinsäurekonjugate in dem Gemisch gebunden wird,
(d) das Entfernen der nicht umgesetzten Nucleinsäurekonjugate unter Verwendung eines Bindungselements, das die Fanggruppe bindet,
wodurch eine gereinigte Bibliothek von Nucleinsäurekonjugaten erzeugt wird, die verschiedene Kombinationen von chemischen Gruppierungen und weiteren chemischen Gruppierungen umfassen,
wobei das Verfahren gegebenenfalls das Wiederholen der Schritte (a) bis (d) und außerdem Folgendes umfasst:
e) das Aufteilen der gereinigten Bibliothek in eine Vielzahl von Pools,
f) das Kontaktieren jedes Pools mit einer anderen weiteren chemischen Gruppierung, wobei die weitere chemische Gruppierung in der Lage ist, mit den chemischen Gruppierungen der Nucleinsäurekonjugate in dem Pool zu reagieren, um kovalente Bindungen zu bilden,
g) das Umsetzen der Nucleinsäurekonjugate in jedem Pool mit der weiteren chemischen Gruppierung zur Herstellung von Reaktionsprodukten, die Nucleinsäurekonjugatprodukte umfassen, wobei die Nucleinsäurekonjugatprodukte Kombinationen einer chemischen Gruppierung und einer weiteren chemischen Gruppierung umfassen, wobei jede Kombination an einen Nucleinsäurestrang konjugiert ist, und entweder
(i)
(h) das Kombinieren der Reaktionsprodukte in den Pools zur Herstellung einer Bibliothek von Nucleinsäurekonjugaten, die Kombinationen von chemischen Gruppierungen und weiteren chemischen Gruppierungen umfassen, wobei jede Kombination an einen Nucleinsäurestrang konjugiert ist,
(i) das Kontaktieren der Bibliothek mit einem Verkappungsmolekül, das eine Fanggruppe umfasst, wobei das Verkappungsmolekül mit nicht umgesetzten Nucleinsäurekonjugatreaktanten reagiert, um kovalente Bindungen zu bilden, und
(j) das Entfernen nicht umgesetzter Nucleinsäurekonjugatreaktanten aus der Bibliothek unter Verwendung eines Bindungselements, das an die Fanggruppe bindet, um eine gereinigte Bibliothek von Nucleinsäurekonjugatprodukten herzustellen, die eine vielfältige Population von Kombinationen von chemischen Gruppierungen und weiteren chemischen Gruppierungen umfasst, wobei jede Kombination an einen Nucleinsäurestrang konjugiert ist; oder
(ii)
(h) das Kontaktieren der Reaktionsprodukte in jedem Pool mit einem Verkappungsmolekül, das eine Fanggruppe umfasst, wobei das Verkappungsmolekül mit nicht umgesetzten Nucleinsäurekonjugatreaktanten reagiert, um kovalente Bindungen zu bilden, und
(i) das Entfernen nicht umgesetzter Nucleinsäurekonjugatreaktanten aus jedem Pool unter Verwendung eines Bindungselements, das an die Fanggruppierung bindet, um eine Population gereinigter Nucleinsäurekonjugatprodukte in jedem Pool herzustellen, und
(j) das Kombinieren der Populationen gereinigter Nucleinsäurekonjugatprodukte in den Pools zur Herstellung einer Bibliothek von Nucleinsäurekonjugatprodukten, die Kombinationen von chemischen Gruppierungen und weiteren chemischen Gruppierungen umfassen, wobei jede Kombination an einen Nucleinsäurestrang konjugiert ist, und
wobei das Verfahren gegebenenfalls das Wiederholen der Schritte (e) bis (j) umfasst, um eine oder mehrere zusätzliche chemische Gruppierungen hinzuzufügen, um eine gereinigte Bibliothek herzustellen, die eine vielfältige Population von Kombinationen von chemischen Gruppierungen und zwei oder mehr zusätzlichen chemischen Gruppierungen umfasst, wobei jede Kombination an einen Nucleinsäurestrang konjugiert ist.

18. Verfahren zur Herstellung einer Nucleinsäurebibliothek, das Folgendes umfasst:
das Bereitstellen einer Vielzahl von Reaktionspools, die jeweils Nucleinsäurestränge umfassen, die eine erste reaktive Gruppe umfassen,
das Kontaktieren jedes Pools mit einer anderen chemischen Gruppierung, die in der Lage ist, mit der ersten reaktiven Gruppe der Nucleinsäurestränge zu reagieren, um kovalente Bindungen zu bilden,
das Umsetzen der Nucleinsäurestränge in jedem Pool mit den chemischen Gruppierungen zur Bildung von Reaktionsprodukten, die Nucleinsäurekonjugate umfassen, die die chemischen Gruppierungen umfassen, und entweder
(i)
(a) das Kombinieren der Reaktionsprodukte in den Pools zur Herstellung einer Bibliothek von Nucleinsäurekonjugaten, die Nucleinsäurestränge gebunden an verschiedene chemische Gruppierungen umfassen,
(b) das Kontaktieren der Bibliothek mit einem Verkappungsmolekül, das eine Fanggruppe umfasst, wobei das Verkappungsmolekül mit den ersten reaktiven Gruppen der nicht umgesetzten Nucleinsäurestränge reagiert, um kovalente Bindungen zu bilden, und
(c) das Entfernen nicht umgesetzter Nucleinsäurestränge aus der Bibliothek unter Verwendung eines Bindungselements, das an die Fanggruppierung bindet, um eine gereinigte Bibliothek von Nucleinsäurekonjugaten herzustellen, wobei die Nucleinsäurekonjugate Nucleinsäurestränge umfassen, die an verschiedene chemische Gruppierungen gebunden sind; oder
(ii)
(a) das Kontaktieren der Reaktionsprodukte in jedem Pool mit einem Verkappungsmolekül, das eine Fanggruppe umfasst, wobei das Verkappungsmolekül mit der ersten reaktiven Gruppe von nicht umgesetzten Nucleinsäuresträngen reagiert, um kovalente Bindungen zu bilden,
(b) das Entfernen nicht umgesetzter Nucleinsäurestränge unter Verwendung eines Bindungselements, das an die Fanggruppierung bindet, um eine gereinigte Population von Nucleinsäurekonjugaten in jedem Pool zu erzeugen, und
(c) das Kombinieren der gereinigten Populationen zur Herstellung einer gereinigten Bibliothek von Nucleinsäurekonjugaten, wobei die Nucleinsäurekonjugate Nucleinsäurestränge umfassen, die an verschiedene chemische Gruppierungen gebunden sind.

## Revendications

1. Procédé de production d'une population purifiée de produits réactionnels conjugués d'acides nucléiques comprenant ;
la fourniture d'éléments réactionnels qui comprennent des produits réactionnels conjugués d'acides nucléiques et des réactifs d'acides nucléiques ou de conjugués d'acides nucléiques, dans lequel lesdits réactifs comprennent un premier groupe réactif,
la mise en contact des éléments réactionnels avec une molécule de coiffage comprenant un groupe de capture, dans lequel la molécule de coiffage réagit avec le premier groupe réactif pour former une liaison covalente, en fixant ainsi le groupe de capture aux réactifs d'acides nucléiques ou de conjugués d'acides nucléiques dans les éléments réactionnels,
l'élimination des réactifs d'acides nucléiques ou de conjugués d'acides nucléiques des éléments réactionnels en utilisant un élément de liaison qui lie le groupe de capture,
en produisant ainsi une population purifiée de produits conjugués d'acides nucléiques.

2. Procédé selon la revendication 1 dans lequel les éléments réactionnels sont fournis par un procédé comprenant ;
la réaction d'une population de réactifs d'acides nucléiques ou de conjugués d'acides nucléiques comportant un premier groupe réactif avec une population de groupements chimiques qui réagissent avec le premier groupe réactif pour produire des éléments réactionnels comprenant des produits conjugués d'acides nucléiques qui contiennent le groupement chimique et les réactifs d'acides nucléiques ou de conjugués d'acides nucléiques n'ayant pas réagi.

3. Procédé selon la revendication 1 ou la revendication 2 dans lequel les éléments réactionnels comprennent des brins d'acides nucléiques comportant un premier groupe réactif, facultativement dans lequel le premier groupe réactif est au niveau d'une terminaison des brins d'acides nucléiques.

4. Procédé selon la revendication 1 ou la revendication 2 dans lequel les éléments réactionnels comprennent des réactifs de conjugués d'acides nucléiques comportant un premier groupe réactif, facultativement dans lequel les réactifs de conjugués d'acides nucléiques comprennent un brin d'acide nucléique conjugué à au moins un groupement chimique et dans lequel le groupement chimique conjugué comprend le premier groupe réactif.

5. Procédé selon la revendication 4 dans lequel les réactifs de conjugués d'acides nucléiques comprennent un brin d'acide nucléique lié à n groupements chimiques, où n vaut 1 à 10, et les produits conjugués d'acides nucléiques comprennent une molécule d'acide nucléique liée à n+1 groupements chimiques.

6. Procédé selon l'une quelconque des revendications 1 à 5 dans lequel les réactifs d'acides nucléiques ou de conjugués d'acides nucléiques sont éliminés par mise en contact des éléments réactionnels avec l'élément de liaison et séparation des produits réactionnels des réactifs d'acides nucléiques ou de conjugués d'acides nucléiques qui sont liés à l'élément de liaison.

7. Procédé selon l'une quelconque des revendications 1 à 6 dans lequel l'élément de liaison est immobilisé sur un support solide.

8. Procédé selon l'une quelconque des revendications 1 à 7 dans lequel le groupe de capture comprend de la biotine ou un dérivé de biotine.

9. Procédé selon la revendication 8 dans lequel le dérivé de biotine est une desthiobiotine ou une iminobiotine.

10. Procédé selon l'une quelconque des revendications 8 à 9 dans lequel l'élément de liaison comprend une protéine de liaison à la biotine.

11. Procédé selon la revendication 10 dans lequel la protéine de liaison à la biotine est une streptavidine ou une avidine.

12. Procédé selon l'une quelconque des revendications 2 à 8 dans lequel le groupement chimique comprend un deuxième groupe réactif qui réagit avec le premier groupe réactif pour former une liaison covalente et la molécule de capture comprend un troisième groupe réactif qui réagit avec le premier groupe réactif pour former une liaison covalente.

13. Procédé selon la revendication 12, dans lequel :
(a) le premier groupe réactif est une amine et les deuxième et troisième groupes réactifs sont des dérivés d'acide carboxylique activés, ou vice versa ;
(b) le premier groupe réactif est un aldéhyde et l'un des deuxième et troisième groupes réactifs est une alcoxyamine ou une 1,2-mercaptoamine et l'autre est une amine primaire ; ou
(c) le premier groupe réactif est un groupe chloroacétyle et l'un des deuxième et troisième groupes réactifs est un groupe nucléophile, tel qu'un thiol ou un phosphorothioate, et l'autre est une amine primaire.

14. Procédé selon l'une quelconque des revendications 1 à 13 comprenant en outre
(a) la réaction de la population purifiée de conjugués d'acides nucléiques avec une population de groupements chimiques additionnels réactifs avec le groupement chimique du conjugué d'acide nucléique, pour produire des produits réactionnels comprenant des produits conjugués d'acides nucléiques comprenant le groupement chimique additionnel et des réactifs de conjugués d'acides nucléiques,
(b) la mise en contact du mélange avec une molécule de coiffage qui comprend un groupe de capture, dans lequel le groupe de coiffage réagit avec le groupement chimique des réactifs de conjugués d'acides nucléiques dans les produits réactionnels pour former des liaisons covalentes, en fixant ainsi le groupe de capture aux réactifs de conjugués d'acides nucléiques, et,
(c) l'élimination des réactifs de conjugués d'acides nucléiques n'ayant pas réagi des produits réactionnels en utilisant un élément de liaison qui lie le groupe de capture,
en produisant ainsi une population purifiée de produits conjugués d'acides nucléiques comprenant le groupement chimique additionnel,
facultativement dans lequel le procédé comprend la répétition des (a) à (c) une ou plusieurs fois pour produire une population purifiée de produits conjugués d'acides nucléiques comprenant deux groupements chimiques additionnels ou plus.

15. Procédé de préparation d'une banque chimique d'acides nucléiques comprenant :
(a) la fourniture d'une population de brins d'acides nucléiques, chaque brin comprenant un premier groupe réactif,
(b) la réaction des brins d'acides nucléiques avec une population diversifiée de groupements chimiques capables de réagir avec le premier groupe réactif pour produire des produits réactionnels comprenant une population diversifiée de conjugués d'acides nucléiques qui comprennent des brins d'acides nucléiques conjugués aux groupements chimiques,
(c) la mise en contact des produits réactionnels avec une molécule de coiffage qui comprend un groupe de capture, dans lequel la molécule de coiffage réagit avec le premier groupe réactif des brins d'acides nucléiques n'ayant pas réagi dans le mélange pour former des liaisons covalentes, en fixant ainsi le groupe de capture aux brins d'acides nucléiques n'ayant pas réagi,
(d) l'élimination des brins d'acides nucléiques n'ayant pas réagi des produits réactionnels en utilisant un élément de liaison qui lie le groupe de capture, en produisant ainsi une banque purifiée de conjugués d'acides nucléiques.

16. Procédé selon la revendication 15 dans lequel chaque brin d'acide nucléique comprend une séquence de codage qui code pour le groupement chimique qui y est conjugué.

17. Procédé selon la revendication 15 ou 16 comprenant en outre :
(a) la fourniture d'une banque purifiée de conjugués d'acides nucléiques comprenant des brins d'acides nucléiques et des groupements chimiques,
(b) la réaction de la banque avec une population diversifiée de groupements chimiques additionnels pour produire des produits réactionnels comprenant une population diversifiée de produits conjugués d'acides nucléiques, chaque produit conjugué d'acide nucléique comprenant une combinaison d'un groupement chimique et d'un groupement chimique additionnel,
(c) la mise en contact des produits réactionnels avec une molécule de coiffage qui comprend un groupe de capture, de manière à ce que la molécule de coiffage réagisse avec les conjugués d'acides nucléiques n'ayant pas réagi pour former des liaisons covalentes, en fixant ainsi le groupe de capture aux conjugués d'acides nucléiques n'ayant pas réagi dans le mélange,
(d) l'élimination des conjugués d'acides nucléiques n'ayant pas réagi en utilisant un élément de liaison qui lie le groupe de capture,
en produisant ainsi une banque purifiée de conjugués d'acides nucléiques comprenant différentes combinaisons de groupements chimiques et de groupements chimiques additionnels,
facultativement dans lequel le procédé comprend la répétition de (a) à (d) et comprend en outre :
e) la division de la banque purifiée en une pluralité de pools,
f) la mise en contact de chaque pool avec un groupement chimique additionnel différent, le groupement chimique additionnel étant capable de réagir avec les groupements chimiques des conjugués d'acides nucléiques dans le pool pour former des liaisons covalentes,
g) la réaction des conjugués d'acides nucléiques dans chaque pool avec le groupement chimique additionnel pour former des produits réactionnels comprenant des produits conjugués d'acides nucléiques, les produits conjugués d'acides nucléiques comprenant des combinaisons d'un groupement chimique et d'un groupement chimique additionnel, chaque combinaison étant conjuguée à un brin d'acide nucléique, et soit ;
(i)
(h) la combinaison des produits réactionnels dans les pools pour produire une banque de conjugués d'acides nucléiques comprenant des combinaisons de groupements chimiques et de groupements chimiques additionnels, chaque combinaison étant conjuguée à un brin d'acide nucléique,
(i) la mise en contact de la banque avec une molécule de coiffage comprenant un groupe de capture, dans lequel la molécule de coiffage réagit avec les réactifs de conjugués d'acides nucléiques n'ayant pas réagi pour former des liaisons covalentes, et
(j) l'élimination des réactifs de conjugués d'acides nucléiques n'ayant pas réagi de la banque en utilisant un élément de liaison qui se lie au groupement de capture pour produire une banque purifiée de produits conjugués d'acides nucléiques comprenant une population diversifiée de combinaisons de groupements chimiques et de groupements chimiques additionnels, chaque combinaison étant conjuguée à un brin d'acide nucléique ; soit
(ii)
(h) la mise en contact des produits réactionnels dans chaque pool avec une molécule de coiffage comprenant un groupe de capture, dans lequel la molécule de coiffage réagit avec les réactifs de conjugués d'acides nucléiques n'ayant pas réagi pour former des liaisons covalentes, et (i) l'élimination des réactifs de conjugués d'acides nucléiques n'ayant pas réagi de chaque pool en utilisant un élément de liaison qui se lie au groupement de capture pour produire une population de produits conjugués d'acides nucléiques purifiés dans chaque pool, et
(j) la combinaison des populations de produits conjugués d'acides nucléiques purifiés dans les pools pour produire une banque de produits conjugués d'acides nucléiques comprenant des combinaisons de groupements chimiques et de groupements chimiques additionnels, chaque combinaison étant conjuguée à un brin d'acide nucléique, et facultativement dans lequel le procédé comprend la répétition des étapes (e) à (j) pour ajouter un ou plusieurs groupements chimiques additionnels pour produire une banque purifiée comprenant une population diversifiée de combinaisons de groupements chimiques et de deux groupements chimiques additionnels ou plus, chaque combinaison étant conjuguée à un brin d'acide nucléique.

18. Procédé de préparation d'une banque chimique d'acides nucléiques comprenant
la fourniture d'une pluralité de pools réactionnels, chacun comprenant des brins d'acides nucléiques comprenant un premier groupe réactif,
la mise en contact de chaque pool avec un groupement chimique différent capable de réagir avec le premier groupe réactif des brins d'acides nucléiques pour former des liaisons covalentes,
la réaction des brins d'acides nucléiques dans chaque pool avec les groupements chimiques pour former des produits réactionnels comprenant des conjugués d'acides nucléiques comprenant les groupements chimiques, et soit ;
(i)
(a) la combinaison des produits réactionnels dans les pools pour produire une banque de conjugués d'acides nucléiques comprenant des brins d'acides nucléiques liés à différents groupements chimiques,
(b) la mise en contact de la banque avec une molécule de coiffage comprenant un groupe de capture, dans lequel la molécule de coiffage réagit avec les premiers groupes réactifs des brins d'acides nucléiques n'ayant pas réagi pour former des liaisons covalentes, et
(c) l'élimination des brins d'acides nucléiques n'ayant pas réagi de la banque en utilisant un élément de liaison qui se lie au groupement de capture pour produire une banque purifiée de conjugués d'acides nucléiques, lesdits conjugués d'acides nucléiques comprenant des brins d'acides nucléiques liés à différents groupements chimiques ; soit
(ii)
(a) la mise en contact des produits réactionnels dans chaque pool avec une molécule de coiffage comprenant un groupe de capture, dans lequel la molécule de coiffage réagit avec les premiers groupes réactifs des brins d'acides nucléiques n'ayant pas réagi pour former des liaisons covalentes,
(b) l'élimination des brins d'acides nucléiques n'ayant pas réagi en utilisant un élément de liaison qui se lie au groupement de capture pour produire des populations purifiées de conjugués d'acides nucléiques dans chaque pool, et
(c) la combinaison des populations purifiées pour produire une banque purifiée de conjugués d'acides nucléiques, lesdits conjugués d'acides nucléiques comprenant des brins d'acides nucléiques liés à différents groupements chimiques.
